(19) Europäisches Patentamt / European Patent Office / Office européen des brevets

(11) **EP 3 717 903 B1**

(12) **FASCICULE DE BREVET EUROPEEN**

(45) Date de publication et mention de la délivrance du brevet:
**03.01.2024 Bulletin 2024/01**

(21) Numéro de dépôt: **18811025.8**

(22) Date de dépôt: **30.11.2018**

(51) Classification Internationale des Brevets (IPC):
**G01N 33/487** (2006.01)   **G01N 33/49** (2006.01)
**G01N 21/31** (2006.01)

(52) Classification Coopérative des Brevets (CPC):
**G01N 21/31**

(86) Numéro de dépôt international:
**PCT/EP2018/083173**

(87) Numéro de publication internationale:
**WO 2019/106159 (06.06.2019 Gazette 2019/23)**

(54) **PROCÉDÉ D'ÉTUDE SPECTRALE D'UN FLUIDE BIOLOGIQUE**

VERFAHREN ZUR SPEKTROSKOPISCHEN UNTERSUCHUNG EINER BIOLOGISCHEN FLÜSSIGKEIT

METHOD OF SPECTRAL INVESTIGATION OF A BIOLOGICAL FLUID

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.11.2017 FR 1761487**

(43) Date de publication de la demande:
**07.10.2020 Bulletin 2020/41**

(73) Titulaires:
• **INSTITUT NATIONAL DE LA SANTE ET DE LA RECHERCHE
MEDICALE (INSERM)
75013 Paris (FR)**
• **Université Paris-Est Créteil Val de Marne
94010 Creteil Cedex (FR)**
• **Etablissement Français du Sang
93210 La Plaine Saint Denis (FR)**
• **Assistance Publique - Hôpitaux de Paris
75004 Paris (FR)**

(72) Inventeurs:
• **KIGER, Laurent
75013 Paris (FR)**
• **MARDEN, Michael
93600 Aulnay Sous Bois (FR)**
• **BARTOLUCCI, Pablo
94240 L'Hay Les Roses (FR)**
• **PIRENNE, France
75012 Paris (FR)**

(74) Mandataire: **Icosa
83 avenue Denfert-Rochereau
75014 Paris (FR)**

(56) Documents cités:
**EP-A1- 1 211 505   US-A1- 2007 292 963
US-B1- 9 638 686**

• H J Duiser ET AL: "Iterative model for the calculation of oxyhemoglobin, methemoglobin, and bilirubin in absorbance spectra of cerebrospinal fluid", Clinical chemistry, 1 février 2001 (2001-02-01), page 338, XP055481163, United States Extrait de l'Internet: URL:https://www.researchgate.net/profile/Frederick_Roelandse/publication/12169533_Iterative_model_for_the_calculation_of_oxyhemoglobin_methemoglobin_and_bilirubin_in_absorbance_spectra_of_cerebrospinal_fluid/links/0912f50ffb76e29652000000/Iterative-model-for-the-calculation-of-oxyhemoglobin-methemoglobi

• J Kamal ET AL: "Binding of heme to human serum albumin: steady-state fluorescence, circular dichroism and optical difference spectroscopic studies", Indian journal of biochemistry & biophysics, 1 février 2005 (2005-02-01), pages 7-12, XP055481473, India Extrait de l'Internet: URL:https://pdfs.semanticscholar.org/1102/a0a25429731dc3acb8812ae37cb90d2c6856.pdf

- **P ASCENZI ET AL: "Heme-based catalytic properties of human serum albumin", CELL DEATH DISCOVERY, vol. 1, no. 1, 7 septembre 2015 (2015-09-07), XP055481476, DOI: 10.1038/cddiscovery.2015.25**

**Description**

DOMAINE TECHNIQUE

L'invention concerne un procédé d'étude spectrale d'un fluide biologique à partir d'un spectre d'absorption d'un échantillon du fluide biologique. L'invention concerne également l'utilisation des données spectrales obtenues par le procédé d'étude spectrale pour déterminer la teneur d'une ou plusieurs protéines, en particulier les hémoprotéines ainsi que l'hème et ses dérivés de dégradation. L'invention concerne un certain nombre d'applications au cas de maladies liées à l'hème et à l'hémoprotéine. L'invention concerne également un programme d'ordinateur comportant des instructions pour l'exécution des étapes d'un procédé d'étude spectrale d'un fluide biologique et un support d'enregistrement lisible par un ordinateur sur lequel est enregistré ledit programme d'ordinateur.

ARRIERE-PLAN TECHNOLOGIQUE

Les maladies hémolytiques parmi lesquelles la drépanocytose, les thalassémies, les pathologies membranaires du globule rouge (GR), les déficits enzymatiques (glucose 6-phosphate déshydrogénase ou G6PD), les hémolyses auto-immunes (AHAI), l'hémoglobinurie paroxystique nocturne, certaines infections (paludisme), le syndrome hémolytique et urémique atypique et également les hémolyses iatrogènes post transfusionnelles (DHTR) ou liées à une dysfonction de valve prothétique cardiaque, ou les hémolyses lors d'une circulation du sang extracorporelle (CEC ou ECMO en anglais) entraînent, après la lyse du GR dans le compartiment sanguin une augmentation de l'hémoglobine (Hb) plasmatique et de l'hème sous forme oxydée non protégé par la globine appelé encore hémine qui se lie de façon transitoire à l'albumine sérique (AS) et de manière quasi-irréversible à l'hémopexine (Hx). La présence d'hème peut être envisagée également dans les hémolyses du nouveau-né du fait de leur faible concentration plasmatique en haptoglobine responsable de la clairance de l'Hb plasmatique ainsi que dans le suivi du traitement des porphyries par injection intraveineuse de solution d'hème.

D'autres pathologies peuvent être mentionnées comme le diabète, le spesis et autres syndromes inflammatoires, l'amylose, les maladies neuro-dégénératives (Alzheimer, Huntington...), les maladies musculaires (relargage de la myoglobine), myopathies.

Par ailleurs, la mesure de l'Hb et surtout de l'hème cytotoxique à des concentrations élevées par rapport à l'homéostasie dans la cellule mais aussi dans un milieu extra-cellulaire (fluides biologiques) ou dans des agrégats moléculaires et cellulaires anormaux (dépôts athérome ou plaques séniles) reste un paramètre biologique important pour le diagnostic de nombreuses pathologies.

L'hème et l'Hb plasmatiques activent une cascade de réactions.

L'hème et l'Hb plasmatiques génèrent des réactions pro-inflammatoires possiblement via les macrophages et les cellules endothéliales par exemple par la liaison au récepteur TLR4 : celle-ci induit entre autres l'expression de cytokines et du facteur tissulaire proagrégeant et finalement l'adhérence des leucocytes et des globules rouges aux parois vasculaires. L'hème active également le complément par la voie alterne.

L'hème et l'Hb plasmatiques ont également pour effet d'activer les réactions procoagulantes en facilitant la formation du thrombus et/ou les réactions pro-oxydantes en générant des dérivés réactifs de l'oxygène (DRO ou ROS ou radicaux libres) ou du monoxyde d'azote (RNS) via la réaction de Fenton et en participant à l'oxydation des lipides membranaires.

Par ailleurs, l'hème et l'Hb plasmatiques sont responsables de l'apoptose et la nécrose tissulaire de l'endothélium mais également des organes.

En outre, l'hème et l'Hb plasmatiques agissent en cas de présence élevée chronique sur le tonus vasculaire voire induisent des modifications de l'anatomie cardio-vasculaire. L'Hb plasmatique a un effet vasoconstricteur en éliminant le NO produit par la NO synthase endothéliale par l'intermédiaire de sa fonction dioxygénase ($HbFe_2$ en réaction avec $O_2$ et NO donne $HbFe^{3+}$ et $NO^{3-}$). L'hème induit quant à lui un effet vasodilatateur via sa dégradation par l'hème oxygénase endothéliale. En effet, le monoxyde de carbone produit de la réaction de catabolisme de l'hème peut inhiber l'activité NO dioxygénase des globines endothéliales et ainsi potentialiser la voie NO/GMPc/PKG (voie de modulation du tonus vasculaire par production du NO dans les cellules endothéliales induisant la production de GMPc par la guanylate cyclase puis l'activation de la protéine kinase C dans les cellules musculaires lisses et in fine la vasodilatation). Une telle réaction biochimique dépend de l'activité hème oxygénase qui bien que surexprimée par l'hème peut être insuffisante pour cataboliser un excès d'hème entraînant des réactions d'oxydation (ROS) voire d'altération de la membrane cellulaire et in fine une vasoconstriction et une inflammation locale.

L'haptoglobine (Hp) et l'hémopexine (Hx) sont deux protéines de phase aigüe de l'inflammation qui ont un rôle majeur respectivement dans l'élimination de l'Hb et de l'hème plasmatiques par endocytose dans le système réticulo-endothélial et les macrophages/hépatocytes respectivement. Dans le cas d'hémolyses intra-vasculaires chroniques ou suite à une crise hémolytique sévère, la concentration de ces deux protéines diminue jusqu'à sa quasi-déplétion. L'hème, ainsi que l'Hb, peuvent alors exercer des effets toxiques par extravasation à travers la paroi des vaisseaux, accumulation à la surface de l'endothélium, par réaction avec le NO produit par la NO synthase entraînant une vasoconstriction locale et participer à de nombreuses atteintes d'organes (rein, foie, rate, coeur, cerveau, poumons). L'activité de l'hème oxygénase sera donc cruciale pour assurer le catabolisme de l'hème et ainsi limiter les réactions cytotoxiques liées à l'Hb/hème.

Si le dosage de l'Hb plasmatique est accessible par spectroscopie et souvent corrélé aux marqueurs d'hémolyse tels que le lactate déshydrogénase (LDH) et l'haptoglobine (ces derniers marqueurs de l'hémolyse n'étant néanmoins pas toujours fiables), celui de l'hème est techniquement difficile du fait de la présence de la bilirubine et de l'Hb généralement en plus forte concentration dans le plasma, ce qui gêne la détection de l'hème. Très peu de données fiables dans la littérature sont rapportées et souvent l'hème plasmatique fait référence à l'Hb. Outre ce constat, sa mesure reste un enjeu majeur pour évaluer les risques post-hémolyse des patients en crise hémolytique. Sa présence est intimement liée à la déplétion de l'haptoglobine et de l'hémopexine mais également à une réaction d'oxydation de l'Hb qui conduit à la production de methémoglobine (metHb) puis à la dissociation de la globine et de l'hème qui devient alors libre. La présence d'hème plasmatique révèle également la présence de débris membranaires, microparticules et autres fantômes d'hématies du fait de l'hémolyse qui peuvent être impliqués dans des réactions pathologiques. La dégradation de l'hème hors prise en charge par l'hème oxygénase aura pour conséquence un relargage du fer. Ce métal de transition altère les structures cellulaires via des réactions d'oxydation avec l'$O_2$ (ROS) et les phospholipides, en particulier, si les protéines de stockage et transfert du fer sont absentes. La dégradation de l'hème dans le compartiment vasculaire sera donc une composante du fer sérique libre toxique et non complexé à l'hème (plasmatique et globinique) d'autant que le coefficient à la transferrine est élevé ; le fer pourra également extravaser lié à l'hème avant d'être relargué dans les tissus.

Enfin, il est à noter qu'une composante de l'hème plasmatique peut refléter la dysérythropoïèse avec l'accumulation d'hème dans la moelle induite par une hémolyse excessive et par la surcharge en fer dans l'organisme (trop forte incorporation intestinale).

Du fait de la toxicité importante de l'hème libre dans le plasma et/ou de l'hème lié à l'albumine sérique (AS-hème) via des réactions d'oxydation avec les ROS (Reactive oxygen species), RNOS (reactive nitrogen oxide species) et les nombreuses molécules en interaction avec l'AS, de leur présence presque certaine dans de nombreuses situations pathologiques et de l'absence actuelle de méthode de mesure fiable, il existe un besoin important de mettre au point un procédé facile à mettre en oeuvre d'étude spectrale du plasma sanguin qui puisse être utilisé pour déterminer la teneur en hème total dans le plasma sanguin qui comprend la globine (Hb), l'hème plasmatique et toutes autres formes anormalement présentes formant un complexe avec l'hème. Les produits de dégradation/oxydation de l'hème sont des biomarqueurs importants des hémolyses intratissulaire et intra-vasculaire et du métabolisme hépatique voire endothélial ou encore placentaire dans le cadre de la prééclampsie. La bilirubine non conjuguée et conjuguée sont deux pigments importants du plasma. Bien que la forme conjuguée soit plus soluble, la bilirubine aurait un rôle protecteur antioxydant et antihypertenseur sauf en cas d'hyperbilirubinémie (ictères sévères quand la bilirubine est supérieure à la capacité de liaison de l'AS avec par exemple atteintes cérébrales) c'est pourquoi les inventeurs ont également développé sa mesure spectrale. Les mesures initialement développées sur le plasma sanguin peuvent être appliquées à d'autres échantillons et fluides biologiques tels que : les urines (dans lesquelles il est possible de mettre en évidence l'hématurie/hémoglobinurie et/ou les atteintes rénales et hépatiques ainsi que les dérivés de dégradation de la bilirubine après oxydation tels que l'urobiline et la stercobiline), les liquides interstitiels (dans lesquels il est possible de mettre en évidence des ascites en présence d'hémolyse), le liquide amniotique (dans lesquels il est possible de mettre en évidence des anémies hémolytiques les produits de dégradation de l'Hb lors des prééclampsies), la lymphe, le liquide céphalo rachidien mais également dans des échantillons biologiques à visée transfusionnelle comme le sang ou ses dérivés comme le plasma sanguin (produits sanguins labiles ou PSL), la culture cellulaire.

Le document H J Duiser et al: "itérative model for the calculation of oxyhemoglobin, methemoglobin, and bilirubin in absorbance spectra of cerebrospinal fluid", Clinical Chemistry, États Unis, pages 338-341, 1 février 2001 divulgue un procédé itératif pour la détermination d'une teneur d'au moins une protéine présente dans un fluide biologique à partir d'un spectre d'absorption mesuré.

## RESUME DE L'INVENTION

La présente invention vise à proposer un nouveau procédé d'étude spectrale d'un fluide biologique à partir d'un spectre d'absorption d'un échantillon du fluide biologique.

Il est ainsi proposé un procédé d'étude spectrale d'un fluide biologique comprenant les étapes de :

a) obtenir un spectre d'absorption SA d'un échantillon du fluide biologique ;
b) soustraire audit spectre d'absorption une composante spectrale associée à l'oxyhémoglobine ($HbO_2$) afin d'obtenir un spectre calculé intermédiaire SC1 ;

c) soustraire audit spectre calculé intermédiaire SC1 une composante spectrale associée à la methémoglobine (metHb) afin d'obtenir un spectre calculé intermédiaire SC2;

d) soustraire audit spectre calculé intermédiaire SC2 une composante spectrale associée à la bilirubine afin d'obtenir un spectre calculé intermédiaire SC3;

e) soustraire audit spectre calculé intermédiaire SC3 une composante spectrale associée à l'hème lié à l'albumine sérique (Hème-AS) afin d'obtenir un spectre calculé intermédiaire SC4; et éventuellement soustraire audit spectre calculé intermédiaire SC4 une composante spectrale associée à une ou plusieurs autres protéines et/ou molécules biologiques présentes dans le fluide biologique.

Par exemple, une autre protéine peut être l'hème liée à l'hémopexine (hème Hx).

Alternativement, en l'absence de formes spectrales rares et atypiques, il est possible de simuler le spectre SC3 à l'étape e) comme une combinaison linéaire des spectres de référence associés à l'hème-AS et l'hème-Hx.

Il est également proposé l'utilisation des données spectrales obtenues par le procédé précédent pour déterminer la teneur d'une ou plusieurs protéines présentes dans un fluide biologique choisie(s) parmi $HbO_2$, MetHb, bilirubine, hème-AS, hème-Hx, Hb carboxylée (HbCO), un biomarqueur du catabolisme de l'hème par l'hème oxygénase, la myoglobine, les porphyrines, les produits de dégradation de la bilirubine tels que les produits de dégradation de la bilirubine par l'hème-AS en présence de ROS ($H_2O_2$) (isomères mono-pyrroles (Heme BOXes), di-pyrroles (PDPs) et la biliverdine), dans les urines la porphobiline, le stercobiline et l'urobiline.

L'utilisation des données spectrales obtenues par le procédé précédent permet d'envisager de déterminer la teneur d'autres pigments biologiques est envisageable en cas du dérèglement de la fonction d'un organe ou autres atteintes viscérales entraînant par exemple une cytolyse. Enfin, la quantification ou la pharmaco-cinétique d'une molécule (ou produits de dégradation) médicamenteuse ou à visée diagnostique est également rendue accessibles par étude des propriétés spectrales obtenues après la soustraction des autres composantes classiquement présentes dans le plasma.

La présente description décrit aussi un programme d'ordinateur comportant des instructions pour l'exécution des étapes d'un procédé d'étude spectrale d'un fluide biologique selon l'invention ou d'un procédé d'utilisation selon l'invention lorsque ledit programme est exécuté par un ordinateur.

Il peut être noté que les programmes d'ordinateur mentionnés dans le présent exposé peuvent utiliser n'importe quel langage de programmation, et être sous la forme de code source, code objet, ou de code intermédiaire entre code source et code objet, tel que dans une forme partiellement compilée, ou dans n'importe quelle autre forme souhaitable.

La description concerne, en outre, un support d'enregistrement lisible par un ordinateur sur lequel est enregistré un programme d'ordinateur comprenant des instructions pour l'exécution d'étapes d'un procédé d'étude spectrale d'un fluide biologique tel que précédemment décrit ou d'un procédé d'utilisation tel que précédemment décrit.

Les supports d'enregistrement (ou d'information) mentionnés dans le présent exposé peuvent être n'importe quelle entité ou dispositif capable de stocker le programme. Par exemple, le support peut comporter un moyen de stockage, tel qu'une ROM, par exemple un CD ROM ou une ROM de circuit microélectronique, ou encore un moyen d'enregistrement magnétique, par exemple une disquette (floppy dise) ou un disque dur.

D'autre part, les supports d'enregistrement peuvent correspondre à un support transmissible tel qu'un signal électrique ou optique, qui peut être acheminé via un câble électrique ou optique, par radio ou par d'autres moyens. Le programme selon l'invention peut être en particulier téléchargé sur un réseau de type Internet.

Alternativement, les supports d'enregistrement peuvent correspondre à un circuit intégré dans lequel le programme est incorporé, le circuit étant adapté pour exécuter ou pour être utilisé dans l'exécution du procédé en question.

Il est également proposé :

- un procédé de détermination d'une teneur d'au moins une protéine présente dans un fluide biologique, le procédé de détermination comportant :

  - la mise en oeuvre du procédé d'étude tel que précédemment décrit,
  - l'obtention d'une teneur en oxyhémoglobine à partir du spectre d'absorption SA,
  - l'obtention d'une teneur en methémoglobine à partir du spectre calculé intermédiaire SC1,
  - l'obtention d'une teneur en bilirubine à partir du spectre calculé intermédiaire SC2, et
  - l'obtention d'une teneur en hème lié à l'albumine sérique à partir du spectre calculé intermédiaire SC3 et/ou de l'hème lié à l'hémopexine.

- un procédé de prédiction qu'un sujet est à risque de souffrir d'une maladie liée à l'hème ou à une hémoprotéine, le procédé de prédiction comprenant au moins les étapes consistant à:

  - effectuer les étapes d'un procédé de détermination au moins une teneur en protéine dans un échantillon biologique du sujet, afin d'obtenir des paramètres déterminés, le procédé de détermination étant tel que précédemment décrit, et

- prédire que le sujet est à risque de souffrir de la maladie liée à l'hème (produits de dégradation) ou à une hémoprotéine sur les paramètres déterminés.

- un procédé de diagnostic une maladie liée à l'hème ou à une hémoprotéine, le procédé de diagnostic comprenant au moins les étapes consistant à:

  - effectuer les étapes d'un procédé de détermination au moins une teneur en protéine dans un échantillon biologique du sujet, pour obtenir des teneurs déterminées en protéine, le procédé de détermination étant tel que précédemment décrit, et
  - diagnostiquer la maladie liée à l'hème ou à une hémoprotéine sur la base des teneurs déterminées en protéines.

- un procédé de définition des stades d'une maladie liée à l'hème ou à une hémoprotéine, le procédé de définition comprenant au moins les étapes consistant à:

  - effectuer les étapes d'un procédé de détermination au moins une teneur en protéine dans un échantillon biologique du sujet, pour obtenir des teneurs déterminées en protéine, le procédé de détermination étant tel que précédemment décrit, et
  - définir les stades de la maladie liée à l'hème ou à une hémoprotéine sur la base des teneurs déterminées en protéines.

- un procédé d'identification d'une cible thérapeutique pour prévenir et/ou traiter une maladie liée à l'hème ou à une hémoprotéine, le procédé comprenant au moins les étapes consistant à:

  - effectuer les étapes d'un procédé de détermination d'au moins une teneur en protéine dans un échantillon biologique d'images d'un premier sujet, pour obtenir une première teneur déterminée en protéine, le procédé de détermination étant tel que précédemment décrit et le premier sujet étant un sujet souffrant de la maladie liée à l'hème ou à une hémoprotéine,
  - effectuer les étapes du procédé de détermination d'au moins une teneur en protéine dans un échantillon biologique d'un deuxième sujet, pour obtenir une deuxième teneur déterminée en protéine, le procédé de détermination étant tel que précédemment décrit et le deuxième sujet étant un sujet ne souffrant pas de la maladie liée à l'hème ou à une hémoprotéine,
  - sélectionner une cible thérapeutique sur la base de la comparaison des première et deuxième teneurs déterminées en protéine.

- un procédé d'identification d'un biomarqueur, le biomarqueur étant un biomarqueur diagnostique d'une maladie liée à l'hème ou à une hémoprotéine, un biomarqueur de susceptibilité d'une maladie liée à l'hème ou à une hémoprotéine, un biomarqueur pronostique d'une maladie liée à l'hème ou à une hémoprotéine ou un biomarqueur prédictif en réponse au traitement maladie liée à l'hème ou à une hémoprotéine, le procédé comprenant au moins les étapes consistant à:

  - mettre en oeuvre les étapes d'un procédé de détermination d'au moins une teneur en protéine dans un échantillon biologique d'un premier sujet, pour obtenir une première teneur déterminée, le procédé de détermination étant tel que précédemment décrit et le premier sujet étant un sujet souffrant de la maladie liée à l'hème ou à une hémoprotéine,
  - effectuer les étapes du procédé de détermination d'au moins une teneur en protéine dans un échantillon biologique d'images d'un deuxième sujet, pour obtenir une deuxième teneur déterminée en protéine, le procédé de détermination étant tel que précédemment décrit et le second sujet étant un sujet ne souffrant pas de la maladie liée à l'hème ou à une hémoprotéine, et
  - sélectionner un biomarqueur sur la base de la comparaison des première et deuxième teneurs déterminées en protéine.

- un procédé de criblage d'un composé utile en tant que médicament, le composé ayant un effet sur une cible thérapeutique connue, pour prévenir et/ou traiter une maladie liée à l'hème ou à une hémoprotéine, le procédé comprenant au moins les étapes consistant à :

  - mettre en oeuvre les étapes d'un procédé de détermination d'au moins une teneur en protéine dans un échantillon biologique d'un premier sujet, pour obtenir un premier teneur déterminée en protéine, le procédé de détermination étant tel que précédemment décrit et le premier sujet étant un sujet souffrant de la maladie liée à l'hème ou à une hémoprotéine et ayant reçu le composé,

- effectuer les étapes d'un procédé de détermination d'au moins une teneur en protéine dans un échantillon biologique d'un deuxième sujet, pour obtenir une deuxième teneur déterminée en protéine, le procédé de détermination étant tel que précédemment décrit et le deuxième le sujet étant un sujet souffrant de la maladie liée à l'hème ou à une hémoprotéine et n'ayant pas reçu le composé, et
- sélectionner un composé sur la base de la comparaison des première et seconde teneurs déterminées en protéines.

- un procédé de qualification ou de disqualification de sacs médicaux contenant un échantillon biologique de sujet, le procédé comprenant au moins les étapes consistant à:

  - effectuer les étapes d'un procédé de détermination d'au moins une teneur en protéines dans la poche médicale, pour obtenir des teneurs déterminées en protéines, le procédé de détermination étant tel que précédemment décrit, et
  - qualifier ou disqualifier des poches médicales sur la base des teneurs déterminées en protéines.

- un procédé de suivi d'un traitement contre une maladie liée à l'hème ou à une hémoprotéine chez un sujet souffrant de la maladie liée à l'hème ou à une hémoprotéine et ayant reçu le traitement, le procédé comprenant au moins les étapes consistant à:

  - effectuer les étapes d'un procédé de détermination au moins une teneur en protéine dans un échantillon biologique du sujet, pour obtenir des teneurs déterminées en protéine, le procédé de détermination étant tel que précédemment décrit, et
  - suivre la teneur déterminée en protéines pour suivre un traitement contre une maladie liée à l'hème ou à une hémoprotéine chez un sujet souffrant de la maladie liée à l'hème ou à une hémoprotéine et ayant reçu le traitement.

DESCRIPTION DETAILLEE DE L'INVENTION

Définitions

Au sens de l'invention, le terme « spectre d'absorption » (SA) doit être pris en son sens premier, c'est-à-dire un spectre obtenu par le passage d'une onde électromagnétique, de la lumière en particulier, au travers d'un milieu transparent ou semi-transparent, dans lequel l'absorption affaiblit (voire élimine) les contributions de certaines fréquences, ce qui donne des courbes caractéristiques dudit milieu. Dans le cadre de l'invention, le spectre d'absorption permet de déterminer la concentration de différentes substances présentes dans le fluide biologique en mesurant l'intensité du rayonnement électromagnétique qu'il absorbe à des longueurs d'onde différentes. Le spectre d'absorption peut être obtenu facilement en mettant en oeuvre des appareillages bien connus de l'homme du métier, généralement en utilisant un spectromètre, par exemple un spectromètre piloté par ordinateur.

Au sens de l'invention, le terme « composante spectrale » correspond une composante du spectre d'absorption. Dans le cadre de l'invention, une composante spectrale est associée à une ou plusieurs substances présentes dans le fluide biologique.

Au sens de l'invention, le terme « fluide biologique » correspond à un fluide susceptible de contenir une hémolyse. L'échantillon biologique peut être choisi parmi les urines, les liquides interstitiels, le liquide amniotique, le sang, les dérivés du sang comme par exemple le plasma sanguin. Dans un mode de réalisation particulier, le fluide biologique est le plasma sanguin.

Au sens de l'invention, le terme « teneur » doit être pris en son sens premier, c'est-à-dire la quantité de l'entité d'intérêt. La teneur peut être exprimée en teneur massique, par exemple en picogramme (pg) ou en molarité par exemple en micromole ($\mu$M). De préférence, au sens de l'invention la teneur est une concentration massique exprimée en $\mu$mol/L ou en $\mu$M, de préférence la concentration massique est exprimée en pg/L. de préférence, les dosages des protéines sériques selon l'invention sont généralement exprimées en micro Mole ($\mu$M), en milligramme par décilitre (mg/dL) ou en gramme par litre (g/L).

Le terme « oxyhémoglobine » ou « HbO$_2$ » correspond à la forme oxygénée de l'hémoglobine, c'est-à-dire à l'hémoglobine dont l'hème réduit (Fe$^{2+}$) est lié à l'oxygène.

La methémoglobine (metHb) est une forme de l'hémoglobine dans laquelle le fer de l'hème est à l'état d'oxydation +3 (Fe$^{3+}$) et qui ne lie pas les ligands diatomiques du Fe$^{2+}$ tels que O$_2$, CO ou NO; à l'inverse Fe$^{3+}$ se lie fortement aux

anions CN$^-$ et N$_3^-$ et faiblement à H$_2$O et OH$^-$.

La bilirubine est un produit de dégradation de l'hémoglobine. Elle circule généralement dans le sang liée à l'albumine.

L'hème lié à l'albumine sérique (hème-AS) est oxydé (Fe$^{3+}$) et peut être impliqué dans des réactions de catalyse enzymatique.

L'hème lié à l'hémopexine (Hème-Hx) est oxydé (Fe$^{3+}$) et n'est pas impliqué dans des réactions de catalyse enzymatique du fait de la très forte hexa-coordination du fer (His-Fe-His). Contrairement à la metHb, les ligands ferriques se lient à l'hème-AS et à l'hème-Hx avec une faible affinité

Les « spectres de référence » ou « SR » sont des spectres d'absorption obtenus pour une seule protéine, comme expliqué ci-dessous. Les spectres de référence sont généralement obtenus avant la mise en oeuvre du procédé de l'invention.

Les « spectres calculés » ou « spectres calculés intermédiaires » ou « SC »sont des spectres obtenus après soustraction au spectre d'absorption mesuré d'une espèce spectrale à partir de son spectre de référence pondéré par un facteur de normalisation « K », ratio entre le rapport de l'amplitude du signal de l'espèce déterminée et de sa référence ; à chaque étape du protocole ci-dessous correspond un nouveau spectre calculé au fur et à mesure de la détermination séquentielle de la contribution de chaque espèce spectrale et de leur soustraction au spectre d'absorbance initial SA.

Procédé d'étude spectrale du fluide biologique

Les inventeurs ont mis en évidence un procédé d'étude spectrale particulièrement adapté pour analyser les différents composants d'un fluide biologique. Ce procédé peut notamment être utilisé pour déterminer la teneur d'une ou plusieurs protéines présentes dans le fluide biologique.

Ainsi, il est décrit un procédé d'étude spectrale d'un fluide biologique comprenant les étapes de :

a) obtenir un spectre d'absorption SA d'un échantillon du fluide biologique;
b) soustraire audit spectre d'absorption une composante spectrale associée à l'oxyhémoglobine (HbO$_2$) afin d'obtenir un spectre calculé intermédiaire SC1 ;
c) soustraire audit spectre calculé intermédiaire SC1 une composante spectrale associée à la methémoglobine (metHb) afin d'obtenir un spectre calculé intermédiaire SC2;
d) soustraire audit spectre calculé intermédiaire SC2 une composante spectrale associée à la bilirubine afin d'obtenir un spectre calculé intermédiaire SC3;
e) soustraire audit spectre calculé intermédiaire SC3 une composante spectrale associée à la l'hème lié à l'albumine sérique (Hème-AS) afin d'obtenir un spectre calculé intermédiaire SC4; et éventuellement
f) soustraire audit spectre calculé intermédiaire SC4 une composante spectrale associée à une ou plusieurs autres protéines et/ou molécules biologiques présentes dans le fluide biologique afin d'obtenir un spectre calculé intermédiaire SC5. Dans le plasma une autre protéine présente peut être l'hème-hémopexine (hème-Hx), notamment en cas d'hémolyse.

L'homme du métier saura adapter l'ordre des différentes étapes dans le cas d'un échantillon biologique atypique en suivant l'ordre décroissant de l'intensité des signaux à mesurer sur un même domaine du spectre lumineux et pour deux signaux de même intensité par simulations non linéaires de l'ensemble du signal à partir de leurs dérivées seconde de référence. L'analyse peut être effectuée pour une espèce à faible intensité en comparaison à l'ensemble des espèces du spectre d'absorbance mesuré s'il existe un domaine du spectre de la lumière qui lui est spécifique ou quasi-spécifique, par exemple pour l'HbO$_2$ vers 577 nm.

Etape a)

L'étape a) consiste à obtenir un spectre d'absorption SA d'un échantillon du fluide biologique.

Tel qu'utilisé ici et selon tous les aspects de l'invention, le terme "échantillon" désigne le sang, le sang total frais, le sang périphérique, les cellules mononucléées du sang périphérique (PBMC), le sérum, le plasma ou l'urine.

L'obtention d'un spectre d'absorption SA peut se faire par des méthodes largement connues de l'homme du métier, par exemple avec un spectromètre, de préférence un spectromètre piloté par ordinateur. L'échantillon du fluide biologique peut être analysé immédiatement après le prélèvement ou à un moment ultérieur. Par exemple après conservation à une température comprise entre 10 et 5 °C pendant plusieurs heures. L'échantillon peut également être congelé puis stocké, par exemple à une température allant de -20°C à -80°C. La congélation permet un stockage facile et une analyse ultérieure en limitant au mieux le risque d'altération dans l'échantillon des protéines et/ou des molécules biologiques dosées. L'échantillon peut également être stocké avec un agent qui stabilise la ou les protéines. La préparation de l'échantillon fait partie du travail de routine de l'homme du métier pour obtenir le spectre d'absorption SA de l'échantillon du fluide biologique. Par exemple, l'échantillon peut également être préparé de manière à augmenter la détectabilité de la ou des protéines, par exemple en fractionnant, en diluant et/ou en concentrant l'échantillon.

Dans un mode de réalisation particulier, le spectre de l'étape a) est obtenu à partir d'un échantillon du fluide biologique dilué, afin d'obtenir une Densité Optique (DO) maximale inférieure ou égale à 2, de préférence inférieure ou égale à 1. C'est-à-dire une DO du spectre d'absorption SA de l'échantillon du fluide biologique dont la valeur maximale est inférieure ou égale à 2, de préférence inférieure ou égale à 1.

Dans un mode de réalisation particulier, le spectre de l'étape a) est obtenu dans un domaine de longueurs d'ondes comprenant tout ou partie de l'ultra-violet et tout ou partie du visible, de préférence dans un domaine de longueurs d'ondes allant d'environ 200 nm à environ 1000 nm, de préférence allant de environ 300 nm à environ 700 nm.

Avantageusement, l'échantillon du fluide biologique est tamponné à un pH inférieur à 8, de préférence entre 7,35 et 7,45, de préférence égal à 7,4 (valeur physiologique), car le spectre de la forme metHb dépend du pH avec un pKa ($H_2O$ -> $OH^-$) voisin de 8. L'échantillon peut être tamponné avec une solution tampon, par exemple une solution tampon choisie parmi phosphate de potassium, phosphate de sodium 50 mM, NaCl 50 mM pH 7,4.

Etape b)

L'étape b) consiste à soustraire audit spectre d'absorption SA une composante spectrale associée à l'oxyhémoglobine ($HbO_2$) afin d'obtenir un spectre calculé intermédiaire SC1.

Dans un mode de réalisation particulier, l'étape b) est réalisée en soustrayant la composante spectrale associée à $HbO_2$, obtenue par normalisation d'un spectre de référence SR1 de $HbO_2$, ladite normalisation étant effectuée par rapport audit spectre d'absorption SA en calculant un coefficient de normalisation K1 à une longueur d'onde d'environ 416nm, d'environ 543nm ou d'environ 577nm, de préférence d'environ 577nm.

Par « spectre de référence SR1 de $HbO_2$ », on entend un spectre d'absorption d'un échantillon de $HbO_2$, c'est-à-dire un échantillon comprenant uniquement ou quasi uniquement $HbO_2$. Des échantillons comprenant uniquement ou quasi uniquement $HbO_2$ peuvent être obtenus à partir de globules rouges d'un sujet sain non-fumeur lavés en sérum physiologique et après lyse dans un tampon phosphate 5-10 mM à pH 7,4 puis centrifugés à 15000 g pour récupérer l'$HbO_2$ dans le surnageant qui peut être finalement purifiée sur une colonne chromatographique d'exclusion de taille et/ou d'échange d'ions.

Avantageusement, l'échantillon comprenant uniquement ou quasi uniquement $HbO_2$ comme protéine est tamponné à un pH inférieur à 8, de préférence entre 7,35 et 7,45, de préférence égal à 7,4, de préférence à un pH identique au pH de l'échantillon du fluide biologique.

Avantageusement, le coefficient de normalisation K1 est calculé par détermination du rapport entre les valeurs des dérivées secondes du spectre d'absorption SA et du spectre de référence SR1 à une longueur d'onde d'environ 416 nm, d'environ 540 nm ou d'environ 577 nm, de préférence d'environ 577 nm.

Par le terme « environ », il est entendu que la longueur d'onde vaut environ la valeur X lorsque la longueur d'onde est supérieure ou égale à X -1 nm et inférieure ou égale à X+1 nm.

Ainsi, SC1 peut être obtenu de la manière suivante à une longueur d'onde I d'environ 416 nm, d'environ 543 nm ou d'environ 577 nm, de préférence d'environ 577 nm :

$$SC1 = SA - K1\, x\, SR1$$

Où :

- SR1 désigne le spectre de référence de $HbO_2$ ;
- K1 est donnée par l'expression suivante :

$$K1 = (\frac{d^2SA}{d\lambda^2}) / (\frac{d^2SR1}{d\lambda^2})$$

avec $d^2SA / d\lambda^2$ la dérivée seconde du spectre d'absorption SA à une longueur d'onde $\lambda$ donnée et $d^2SR1/ d\lambda^2$ est dérivée seconde du spectre de référence de $HbO_2$

Généralement, le coefficient de normalisation K1 tient compte du facteur de dilution du fluide biologique.

$HbO_2$ est l'oxyhémoglobine A ($HbAO_2$), l'oxyhémoglobine F ($HbFO_2$), l'oxyhémoglobine A2 ($HbA2O_2$) et les forme glyquées. En général, $HbO_2$ est $HbAO_2$ chez l'adulte et l'$HbO_2$ est un mélange $HbAO_2$ et $HbFO_2$ chez le nouveau-né ou chez les patients avec des variantes de l'Hb S, C, E pour les plus répandues.

Etape c)

L'étape c) consiste à soustraire audit spectre calculé intermédiaire SC1 une composante spectrale associée à la methémoglobine (metHb) afin d'obtenir un spectre calculé intermédiaire SC2.

Avantageusement, la composante spectrale associée à la methémoglobine (metHb) est obtenue à partir du:

- spectre d'absorption de l'échantillon (SA),
- spectre d'absorption de l'échantillon traité au KCN (SA$^{KCN}$),
- spectre de référence de metHb (SR2), et
- spectre de référence de metHb SR2 traité au KCN.

Le spectre différentiel SD égal à SA-SA$^{KCN}$, ci-après SD$^{KCN}$ est obtenu par soustraction des données de DO SA-SA$^{KCN}$ correspondantes aux mêmes longueurs d'onde, de préférence par soustraction : $DO_{max} - DO_{min}$.

Avantageusement, le coefficient de normalisation K2 est calculé par détermination du rapport SD$^{KCN}$ avec un spectre différentiel de référence SDR$^{KCN}$ obtenu à partir d'un échantillon comprenant uniquement ou quasi uniquement metHb en absence de KCN vs saturée en KCN.

Ainsi, SC2 peut être calculé de la manière suivante :

$$SC2 = SC1 - K2 \; x \; SR2$$

Où :

- SR2 est le spectre de référence de la metHb (obtenu à partir d'un échantillon comprenant uniquement ou quasi uniquement metHb en absence de KCN), et

Par ailleurs, il vient :

$$K2 = \frac{SD^{KCN}}{SDR^{KCN}} = \frac{DOmax - DOmin}{DO^R \max - DO^R min}$$

Généralement, $DO_{max}$ et $DO^R_{max}$ (Densité Optique de référence) sont à une longueur d'onde d'environ 404nm, et $DO_{min}$ et $DO^R_{min}$ sont à une longueur d'onde d'environ 424 nm, ou inversement.

Avantageusement, afin d'avoir la plus forte amplitude du signal et ainsi augmenter la sensibilité pour le dosage de la metHb par ajout de KCN et aussi afin d'éliminer un signal parasite provenant d'un déplacement de ligne de base ou une composante diffusionnelle, K2 est calculé en prenant la valeur absolue de la différence entre la DO maximale ($DO_{max}$) et la DO minimale ($DO_{min}$) du spectre SD$^{KCN}$, généralement à environ 404 nm et à environ 424 nm.

Dans un mode de réalisation particulier, le dosage de la teneur en metHb peut se faire par l'analyse de la dérivée seconde de SD$^{KCN}$ en prenant la différence des valeurs de dérivée entre environ 404 nm et environ 424nm ou directement la valeur de la dérivée seconde à une de ces longueurs d'onde.

Alternativement, SC2 peut être calculé de la manière suivante :

$$SC2 = SC1 - KSD2 \; x \; SR2$$

avec

$$KSD2 = [\left(\frac{d^2 SD^{KCN}}{d\lambda^2}\right)_{\lambda=406} - \left(\frac{d^2 SD^{KCN}}{d\lambda^2}\right)_{\lambda=421}]/[\left(\frac{d^2 SDR^{KCN}}{d\lambda^2}\right)_{\lambda=406} - \left(\frac{d^2 SDR^{KCN}}{d\lambda^2}\right)_{\lambda=421}]$$

Par « spectre différentiel SD$^{KCN}$» ou « spectre SD$^{KCN}$» ou « SD$^{KCN}$ », on entend un spectre différentiel représentant la différence spectrale en DO d'un échantillon avant et après ajout de KCN.

Par « spectre différentiel SDR$^{KCN}$» « spectre SDR$^{KCN}$» ou « SDR$^{KCN}$», on entend un spectre différentiel de référence représentant la différence spectrale en DO avant et après ajout de KCN pour un spectre de référence de metHb (SR2).

Par « spectre de référence de metHb » ou « SR2 », on entend un spectre d'absorption d'un échantillon de metHb, c'est-à-dire un échantillon comprenant uniquement ou quasi uniquement la metHb. Un tel échantillon peut être obtenu commercialement ou par la mise en oeuvre de protocoles biochimiques et méthodes classiques de purification. Avantageusement, l'échantillon comprenant uniquement ou quasi uniquement la metHb comme protéine, est tamponné à un pH inférieur à 8, de préférence entre 7,35 et 7,45, de préférence égal à 7,4, de préférence à un pH identique au pH de l'échantillon du fluide biologique.

Dans un mode de réalisation particulier, l'étape c) est réalisée en soustrayant la composante spectrale associée à la metHb, obtenue par normalisation d'un spectre différentiel standard $SDR^{KCN}$ avec le spectre différentiel mesuré $SD^{KCN}$, ladite normalisation étant effectuée par rapport audit spectre d'absorption SA en calculant un coefficient de normalisation K2 à une longueur d'onde comprise entre environ 350 nm et environ 700 nm, de préférence entre environ 380 nm et environ 440 nm.

Avantageusement, le coefficient de normalisation K2 est calculé par détermination du rapport entre les valeurs de $SD^{KCN}$ et $SDR^{KCN}$ en mesurant pour chaque spectre différentiel (i) de préférence la différence entre $DO_{max}$ et $DO_{min}$, et $DO^R_{max}$ et $DO^R_{min}$, (ii) $DO_{max}$ et $DO^R_{max}$, ou (iii) $DO_{min}$ et $DO^R_{min}$.

Généralement, le coefficient de normalisation K2 tient compte du facteur de dilution du fluide biologique.

Alternativement, le coefficient de normalisation KDS2 est calculé sur le même principe excepté qu'au lieu de prendre en compte la différence spectrale d'un spectre avant et après ajout de KCN, c'est la dérivée seconde de la différence spectrale qui est utilisée pour l'échantillon biologique et pour l'échantillon de de metHb.

Outre le KCN, on peut également utiliser le NaCN ou le $NaN_3$ pour doser la metHb, selon le même principe que décrit ci-dessus pour KCN.

A cette étape, seule la metHb est dosée du fait de sa forte affinité pour l'anion $CN^-$ ou $N_3^-$ La concentration des solutions stocks de KCN et de NaCN utilisée peut être par exemple de 50 mM et celle du $NaN_3$ par exemple de 100 mM. Avantageusement, la concentration finale en KCN est comprise entre 100 et 500 $\mu$M, de préférence entre 150 $\mu$M et 250 $\mu$M, de préférence elle est d'environ 200 $\mu$M. Avantageusement, la concentration finale en NaCN est comprise entre 100 à 500 $\mu$M, de préférence entre 150 $\mu$M et 250 $\mu$M, de préférence elle est d'environ 200 $\mu$M. Avantageusement, la concentration finale en $NaN_3$ est comprise entre 0.5 à 1 mM.

Le temps d'incubation de la metHb et le cyanure est d'environ 5 minutes avant son dosage dans le plasma.

Etape d)

L'étape d) consiste à soustraire audit spectre calculé intermédiaire SC2 une composante spectrale associée à la bilirubine afin d'obtenir un spectre calculé intermédiaire SC3.

Dans un mode de réalisation particulier, l'étape d) est réalisée en soustrayant la composante spectrale associée à la bilirubine, obtenue par normalisation d'un spectre de référence SR3 de bilirubine liée à l'AS, ladite normalisation étant effectuée par rapport audit spectre calculé intermédiaire SC2 en calculant un coefficient de normalisation K3 à une longueur d'onde comprise dans tout ou partie de l'ultra-violet et tout ou partie du visible, de préférence une longueur d'onde allant d'environ 350 nm à environ 700 nm.

Avantageusement, le coefficient de normalisation K3 est calculé par détermination du rapport entre les valeurs des dérivées secondes du spectre calculé SC2 et du spectre standard SR3 à une longueur d'onde comprise dans tout ou partie de l'ultra-violet et tout ou partie du visible, de préférence une longueur d'onde allant d'environ 350 nm à environ 700nm, de préférence à une longueur d'onde allant d'environ 440 nm à environ 540 nm, de préférence aux alentours de 501 nm.

Ainsi, SC3 peut être calculé de la manière suivante :

$$SC3 = SC2 - K3 \; x \; SR3 \; \text{à } \lambda \in [440 \text{ nm, } 540 \text{ nm}]$$

Où :

- SR3 est le spectre de référence de la bilirubine, et
- $\lambda \in$ [440 nm, 540 nm] signifie que la longueur d'onde est comprise entre 440nm et 540nm.

Par ailleurs, il vient :

$$K3 = \left(\frac{d^2 SC2}{d\lambda^2}\right) \Big/ \left(\frac{d^2 SR3}{d\lambda^2}\right)$$

Dans un autre mode de réalisation particulier, l'étape d) est réalisée en soustrayant la composante spectrale associée à la bilirubine, obtenue par normalisation d'un spectre de référence SR3 de bilirubine liée à l'AS, ladite normalisation étant effectuée par rapport audit spectre calculé intermédiaire SC1 en calculant un coefficient de normalisation K3 à une longueur d'onde comprise dans tout ou partie de l'ultra-violet et tout ou partie du visible, de préférence une longueur d'onde allant de environ 350 nm à environ 700 nm.

K3 peut donc être calculé ainsi :

$$K3 = \left(\frac{d^2 SC1}{d\lambda^2}\right) \Big/ \left(\frac{d^2 SR3}{d\lambda^2}\right)$$

Par « spectre standard SR3 de bilirubine » ou « spectre standard SR3 », on entend un spectre d'absorption d'un échantillon de bilirubine avec un excès d'albumine sérique (AS), c'est-à-dire un échantillon comprenant uniquement ou quasi uniquement de la bilirubine et un excès d'AS. L'excès d'AS permet d'obtenir de la bilirubine liée à AS ou « bilirubine-AS ». Des échantillons comprenant uniquement ou quasi uniquement de la bilirubine humaine sont disponibles commercialement, par exemple chez Sigma Aldrich. Par exemple, la bilirubine est dissoute dans de la soude NaOH 0.1 N et gardée à l'abri de la lumière avant d'être ajoutée à une solution de AS filtrée sur 0.2 $\mu$M pour un mélange final 1:2 mole à mole. Après 20 minutes d'équilibration à l'abri de la lumière le spectre de bilirubine-AS est enregistré. Avantageusement, l'échantillon, comprenant uniquement ou quasi uniquement de la bilirubine-AS est tamponné à un pH inférieur à 8, de préférence entre 7,35 et 7,45, de préférence égal à 7,4, de préférence à un pH identique au pH de l'échantillon du fluide biologique.

Etape e)

L'étape e) consiste à soustraire audit spectre calculé intermédiaire SC3 une composante spectrale associée à la l'hème lié à l'albumine sérique (Hème-AS) afin d'obtenir un spectre calculé intermédiaire SC4.

Dans un mode de réalisation particulier, l'étape e) est réalisée en soustrayant la composante spectrale associée à la l'hème-AS, obtenue par normalisation d'un spectre de référence SR4 de hème-AS, ladite normalisation étant effectuée par rapport audit spectre calculé intermédiaire SC3 en calculant un coefficient de normalisation K4 à une longueur d'onde comprise dans tout ou partie de l'ultra-violet et tout ou partie du visible, de préférence dans un domaine de longueurs d'ondes allant d'environ 200 nm à environ 800 nm, de préférence allant d'environ 300 nm à environ 700 nm, de préférence allant d'environ 380 nm à environ 460 nm ou entre 600 et 700 nm pour une concentration élevée de l'espèce ou en l'absence de dilution ($\leq$ 1/2) dans un sérum pur ou un plasma prélevé sur citrate pour s'affranchir de la valeur du pH voisine de 7,4.

Avantageusement, le coefficient de normalisation K4 est calculé par détermination du rapport entre les valeurs des dérivées secondes du spectre d'absorption et du spectre de référence SR4 à une longueur d'onde comprise dans tout ou partie de l'ultra-violet et tout ou partie du visible, de préférence une longueur d'onde allant d'environ 350 nm à environ 460 nm.

Ainsi, SC4 peut-être calculé de la manière suivante :

$$SC4 = SC3 - K4 \; x \; SR4 \;\; \text{à } \lambda \in [350 \text{ nm}, 700 \text{ nm}]$$

Où :

- SR4 est un spectre de référence de l'hème-AS, et

$\lambda \in$ [350 nm,700 nm] fait référence à une longueur d'onde comprise entre 350 nm et 700 nm.

Par ailleurs, il vient :

$$K4 = \left(\frac{d^2 SC3}{d\lambda^2}\right) \Big/ \left(\frac{d^2 SR4}{d\lambda^2}\right)$$

Avec $\dfrac{d^2 SC3}{d\lambda^2}$ la dérivée seconde du spectre calculé intermédiaire SC3 et $\dfrac{d^2 SR4}{d\lambda^2}$ la dérivée seconde du spectre de référence SR4

Par « spectre de référence SR4 de l'hème-AS » ou « « spectre de référence SR4 », on entend un spectre d'absorption d'un échantillon d'hème-AS, c'est-à-dire un échantillon comprenant uniquement ou quasi uniquement de l'hème-AS comme protéine. Les échantillons d'hème et d'AS sont disponibles par exemple chez Sigma Aldrich. Par exemple, l'hème est dissout dans une solution de soude NaOH 0,1 N à l'abri de la lumière avant d'être ajouté à une solution d'AS filtrée sur ,.2 μM pour un mélange final 1:2 mole à mole. Après 20 minutes d'équilibration à l'abri de la lumière le spectre d'Hème-AS est enregistré. Avantageusement, l'échantillon, comprenant uniquement ou quasi uniquement de l'hème-AS comme protéine est tamponné à un pH inférieur à 8, de préférence entre 7,35 et 7,45, de préférence égal à 7,4, de préférence à un pH identique au pH de l'échantillon du fluide biologique.

Dans un mode de réalisation particulier, l'étape e) est mise en oeuvre en soustrayant la composante spectrale associée à la l'hème-AS ou à une combinaison linéaire de l'hème-AS et l'hème-Hx, obtenue par normalisation d'un spectre de référence SR4 de l'hème-AS ou de la contribution de deux référence SR4 de l'hème-AS et SR5 de l'hème-Hx, ladite normalisation étant effectuée par rapport audit spectre calculé intermédiaire SC3 en calculant un coefficient de normalisation K4 (ou K4 et K5) à une longueur d'onde comprise dans tout ou partie de l'ultra-violet et tout ou partie du visible, de préférence dans un domaine de longueurs d'ondes allant de environ 200 nm à environ 800 nm, de préférence allant de environ 300 nm à environ 700 nm, de préférence allant de environ 380 nm à environ 450 nm.

Dans un autre mode de réalisation particulier, le coefficient de normalisation K4 ou K4 et K5 sont calculés par détermination du rapport entre les valeurs des dérivées secondes du spectre calculé intermédiaire SC3 et du spectre de référence SR4 ou des spectres de référence SR4 et SR5 (hème-AS et hème-Hx respectivement) à une longueur d'onde comprise dans tout ou partie de l'ultra-violet et tout ou partie du visible, de préférence une longueur d'onde allant d'environ 300 nm à environ 450 nm.

Etape f)

L'étape f) consiste à soustraire audit spectre calculé intermédiaire SC4 une composante spectrale associée à une ou plusieurs autres protéines présentes dans le fluide biologique afin d'obtenir un spectre calculé intermédiaire SC5.

Hormis l'hème-AS, l'espèce la plus attendue pour la liaison de l'hème en cas d'hémolyse faible voire modéré et non chronique ou dans le cas d'une thérapie à base d'hème (porphyries) est l'hème-hémopexine (Hème-Hx).

Alternativement, lorsque la protéine de l'étape f) est Hème-Hx, les étapes e) et f) peuvent être combinés de la manière suivante :

SC4 peut être calculé de la manière suivante à l ε [350,700 nm], de préférence à l ε [360,460 nm]:

$$SC4 = SC3 - K4\,x\,SR4 - K5\,x\,SR5$$

Où SR4 est le spectre de référence de l'hème-AS et SR5 est le spectre de référence hème-Hx.

K4 et K5 sont obtenus en simulant (par simulation, il est entendu une modélisation dans un tel contexte) la dérivée seconde de SC3 comme une combinaison linéaire des deux dérivées seconde de l'hème-AS et de l'hème-Hx pondérées respectivement des coefficients K4 et K5:

$$\left(\frac{d^2 SC3}{d\lambda^2}\right) = K4\left(\frac{d^2 SR4}{d\lambda^2}\right) + K5\,(\frac{d^2 SR5}{d\lambda^2})$$

Avec :

- $\frac{d^2 SC3}{d\lambda^2}$ : la dérivée seconde du spectre calculé intermédiaire SC3,

- $\frac{d^2 SR4}{d\lambda^2}$ : la dérivée seconde du spectre de référence SR4, et

- $\frac{d^2 SR5}{d\lambda^2}$ : la dérivée seconde du spectre de référence SR5.

La simulation peut être effectuée à partir d'une minimisation non linéaire par la technique des moindres carrés ou tout autre type d'algorithme de simulations non linéaires.

Par « spectre de référence SR5 de hème-Hx » ou « « spectre de référence SR5 » ou « SR5 », on entend un spectre d'absorption d'un échantillon d'hème-Hx, c'est-à-dire un échantillon comprenant uniquement ou quasi uniquement de l'hème-Hx comme protéine. Les échantillons d'Hx sont disponibles par exemple chez Sigma Aldrich. Avantageusement, l'échantillon, comprenant uniquement ou quasi uniquement de l'hème-Hx comme protéine est tamponné à un pH inférieur à 8, de préférence entre 7,35 et 7,45, de préférence égal à 7,4, de préférence à un pH identique au pH de l'échantillon du fluide biologique.

Dans un mode de réalisation particulier, la ou les autres protéines présentes dans le fluide biologique sont une ou plusieurs protéines héminiques, par exemple une ou plusieurs protéines choisies parmi HbO$_2$, MetHb, bilirubine, hème-AS, hème-Hx, Hb carboxylée (HbCO), un biomarqueur du catabolisme de l'hème par l'hème oxygénase, la myoglobine, les porphyrines, les produits de dégradation de la bilirubine tels que les produits de dégradation de la bilirubine par l'hème-AS en présence de ROS (H$_2$O$_2$) (isomères mono-pyrroles (Heme BOXes), di-pyrroles (PDPs) et la biliverdine), dans les urines la porphobiline, le stercobiline et l'urobiline.

Il peut également s'agir d'une molécule biologique, par exemple une molécule pharmaceutique administrée à un patient.

Dans un mode de réalisation particulier, l'étape f) est réalisée en soustrayant la composante spectrale associée à la ou aux autres protéines, obtenue par normalisation d'un ou plusieurs spectres de référence SRX (par exemple SR5) de la ou des autres protéines, ladite normalisation étant effectuée par rapport audit spectre calculé intermédiaire (par exemple SC5) en calculant un coefficient de normalisation KX (par exemple K5) à une longueur d'onde que l'homme du métier pourra déterminer pour la ou les autres protéines.

Ainsi, SC5 peut être calculé de la manière suivante à $I \in$ [200,700 nm], par exemple à 420 nm pour HbCO :

$$SC5 = SC4 - K5 \times S5$$

Où :

- SR5 est le spectre référence d'une autre protéine ou molécule biologique, et
- $I \in$ [200,700 nm] signifie que la longueur d'onde est comprise entre 200 nm et 700 nm.

Par ailleurs, il vient ;

$$K5 = \left(\frac{d^2 SC4}{d\lambda^2}\right) / \left(\frac{d^2 SR5}{d\lambda^2}\right)$$

Avec :

- $d^2 SC4 / dl^2$ la dérivée seconde du spectre calculé intermédiaire SC4, et
- $d^2 SR5 / dl^2$ la dérivée seconde du spectre de référence SR5

Dans un mode de réalisation particulier, les spectres de références des étapes b), c), d), e) et f) sont obtenus à partir d'un échantillon des différentes espèces pures, à une Densité Optique (DO) maximale inférieure ou égale à 2, de préférence inférieure ou égale à 1. C'est-à-dire une DO du spectre de référence des espèces dont la valeur maximale est inférieure ou égale à 2, de préférence inférieure ou égale à 1. De manière préférée, les spectres d'absorption mesurés sont analysés à l'aide des spectres de référence dans le violet pour les espèces MetHb, hème-AS et hème-Hx dans le bleu-vert pour la bilirubine non conjuguée et conjuguée et dans le jaune pour l'HbO$_2$ mais également dans le rouge pour l'hème-AS.

Il est bien connu de l'homme du métier qu'il existe une relation de proportionnalité entre l'absorbance et la concentration de l'espèce que l'on cherche à déterminer. Cette relation de proportionnalité répond à l'équation suivante :
A= $\xi$ l c

Avec :

- $\xi$ est le coefficient d'extinction qui dépend de la longueur d'onde ;
- l est la longueur de la cuve (généralement 1 cm), et
- c est la concentration de la solution de référence.

Dans un mode de réalisation particulier, la DO est égale à l'absorbance A, soit :

$$A = DO$$

Dans un mode de réalisation préférée la DO est égale à 1, ce qui correspond à

$$A = DO = 1$$

Il y a donc une proportionnalité entre l'absorbance ou la DO et la concentration avec A = DO.

Par « coefficient d'extinction », on entend la valeur d'absorbance d'une espèce dans un solvant et/ou un soluté à une température donnée normalisée à une concentration et un trajet optique. Le coefficient d'extinction est aussi appelé coefficient d'extinction molaire dont l'unité est généralement donnée en $mM^{-1}$ $cm^{-1}$

Les coefficients d'extinction sont des valeurs normalisées connues de l'homme du métier. Par exemple, les coefficients d'extinction associés aux espèces de référence qui vont permettre de calculer les concentrations de chacune des espèces dans le fluide biologique sont :

- à 577 nm: coefficient d'extinction de 15.5 $mM^{-1}$ $cm^{-1}$ pour $HbO_2$ ;
- à 403-404 nm: coefficient d'extinction de 92 $mM^{-1}$ $cm^{-1}$ pour hème-AS d'origine humaine;
- à 404-424 nm : coefficient d'extinction de 160 $mM^{-1}$ $cm^{-1}$ pour la différence spectrale de la MetHb +/- CN ;
- à 540 nm : coefficient d'extinction de 11.5 $mM^{-1}$ $cm^{-1}$ pour metHb-CN⁻ ;
- à 459 nm +/- 1 nm: coefficient d'extinction de 47 $mM^{-1}$ $cm^{-1}$ pour la bilirubine non conjuguée ;
- à 413-414 nm : coefficient d'extinction de 120 $mM^{-1}$ $cm^{-1}$ pour hème-Hx, et
- à 420 nm: coefficient d'extinction de 190 $mM^{-1}cm^{-1}$ pour HbCO.

Ces coefficients d'extinction permettent de calculer la concentration de l'espèce pour ledit spectre de référence. Par exemple, le coefficient d'extinction relatif à $HbO_2$ permet de calculer la concentration de $HbO_2$ pure relative au spectre SR1.

Ainsi, les concentrations des solutions de référence X sont calculées de la manière suivante :

$$c[sol\ de\ référence\ x] = \frac{A}{\xi\ l}$$

Les valeurs de la DO (proche de 1), $\xi$ et l (1 cm) sont connues.

On en déduit donc C [sol de référence x].

Dans un mode de réalisation préféré, on obtient une DO pour une concentration d'une espèce X, choisi parmi $HbO_2$, metHb, bilirubine, hème-AS, hème-Hx. Pour les autres espèces évoquées certaines sont bien référencées dans la littérature, comme la myoglobine ou certaines hémoprotéines, d'autres devront être calculées après dilution dans le fluide biologique, leur concentration étant estimée par pesée ou enfin à partir d'échantillons biologiques dans lesquels elles sont bien caractérisées ou majoritaires.

De manière non limitative, la concentration ainsi obtenue peut être normalisée à 10$\mu$M, 100$\mu$M ou 1mM ($mM^{-1}$ $cm^{-1}$). Cette normalisation est une normalisation correspondant à une concentration simple d'utilisation ce qui permet de normaliser le spectre de référence à une concentration donnée simple d'utilisation.

Une fois cette concentration connue [c sol ref] normalisée, on calcule la dérivée seconde de l'espèce d'intérêt de l'échantillon de fluide biologique en faisant un rapport d'amplitude entre la dérivée 2nd mesurée et celle de la référence : Par exemple :

$$[HbO_2] = \left(\frac{d^2 SA}{d\lambda^2}\right)_{577\ nm} \cdot f_{PBS} \cdot f_{dilution} / \left(\frac{d^2 SR1}{d\lambda^2}\right)_{577\ nm} \cdot [c\ sol\ ref\ de\ l'HbO_2].$$

Où :

- $f_{PBS}$ est le facteur dilution plasma dans PBS :

$$f_{PBS} = (volume\ échantillon + volume\ PBS)/(volume\ échantillon)$$

et

- $f_{dilution}$ est le facteur dilution du fluide biologique dans un éventuel soluté.

Le rapport d'amplitude entre la dérivée seconde et celle de la référence se faisant à une même longueur d'onde, soit par exemple aux environs de 577 nm pour HbO$_2$.

En particulier, lorsque que le fluide biologique est du plasma à la différence du sérum, le facteur de dilution du sang dans l'anticoagulant est pris en compte. Par exemple le volume d'anticoagulant dans un tube de prélèvement citraté est de 300 µl de solution pour un volume de prélèvement de 3 ml de sang. Le facteur de dilution sera calculé comme suit :

$$f_{dilution} = \frac{(1 - \text{Htc}) \text{ x (volume du prélèvement )}}{(1 - \text{Htc}) \text{ x (volume du prélèvement) } - \text{ volume de soluté)}}$$

Pour un hématocrite Htc de 0,4, le facteur de dilution (facteur multiplicatif) des concentrations des espèces mesurées sera de l'ordre de 1,2. Ce facteur diminuera jusqu'à 1,1 pour des patients avec une anémie hémolytique. Il est à noter que les autres coagulants communément utilisés (héparine ou EDTA) n'induisent pas de dilution. Plus généralement tout facteur de dilution du fait de la présence de soluté dans un tube de prélèvement mais également entre la source biologique et le tube (par exemple lors d'une CEC ou ECMO dans le système extra-corporel) devra être pris en compte. Dans ce dernier cas un marqueur interne stable dans le temps pourra être choisi pour estimer la dilution comme la concentration en protéine totale avant circulation. Ce point est valable pour tous les fluides biologiques. Il est possible également de tenir compte dans certains cas de la perfusion d'un soluté chez un patient et/ou de mesurer par volumétrie l'expansion d'un fluide par exemple pour les urines quand ces données sont disponibles.

De manière préférée, une dilution du fluide biologique est faite a minima au ½, de préférence dans un plasma au 1/6$^{\text{ème}}$ dans une cuve 1 cm (100 µl de fluide biologique -> 500 µl PBS pH 7,4: 50 mM phosphate de potassium 50 mM NaCl) ; La mesure sera ajustée pour des échantillons atypiques en fonction du signal si trop faible ou trop intense en conséquence :

Par exemple,

$$[\text{HbO}_2] = \left(\frac{d^2 SA}{d\lambda^2}\right)_{577\,nm} \cdot f_{PBS} \cdot f_{dilution} / \left(\frac{d^2 SR1}{d\lambda^2}\right)_{577\,nm} \cdot [c\, sol\, ref\, de\, l'HbO_2](\ x\ [\text{HbO}_2\ \text{ref}]$$

x 6 (dilution dans le PBS) x 1.17 (dilution dans le citrate pour un Htc de 0.3).

Soit un facteur de correction de 7 par rapport aux valeurs mesurées.

Finalement la concentration en Hb libre dans le plasma devra être corrigée de la fraction liée irréversiblement à l'haptoglobine. Même si l'HbO$_2$ est généralement majoritaire (>90%) il conviendra de prendre calculer l'Hb totale = HbO$_2$ + MetHb + HbCO. En effet il n'y a pas de différence spectrale entre l'Hb (i.e. HbO$_2$) dans le plasma, libre ou complexée à l'haptoglobine. De plus seule la fraction libre est néfaste pour l'organisme. La mesure de l'haptoglobine si elle est attendue (le plus souvent déplétée chez les patients avec une hémolyse chronique) est réalisée par réaction biochimique avec un anticorps. Elle est donnée en g/L et le poids moléculaire moyen des différentes isoformes liant 2 dimères d'Hb est d'environ 85 kD (soit contenu du poids moléculaire de l'Hb 64.5 kD : 1.3 g d'haptoglobine lie 1 g d'Hb tétramérique).

Utilisation

L'invention concerne également l'utilisation des données spectrales obtenues par le procédé de l'invention pour déterminer la teneur d'une ou plusieurs protéines présentes dans le fluide biologique choisie parmi HbO$_2$, MetHb, la bilirubine conjuguée et non conjuguée, hème-AS, hème-Hx, l'Hb carboxylée (HbCO), un biomarqueur du catabolisme de l'hème par l'hème oxygénase, un produit de dégradation/oxydation de l'hème, un complexe protéine/hème et une molécule biologique.

La détermination de la teneur d'une ou plusieurs protéines présentes dans un fluide biologique peut être mise en oeuvre par un processeur d'un système informatique. Ce système informatique peut comporter des moyens de stockage informatique dans lequel sont stockées des instructions qui, lorsqu'elles sont exécutées par le processeur du système informatique, vont réaliser la comparaison de densité optique (DO).

Généralement, l'homme du métier sait comment régler les paramètres du spectromètre de manière à obtenir directement la teneur en une ou plusieurs protéines présentes dans le fluide biologique.

Le procédé d'étude spectrale permet ainsi d'accéder à une pluralité de teneurs de protéines.

APPLICATIONS

De nombreuses applications de cette méthode déterminante peuvent être envisagées.

Sont notamment développées les applications liées aux maladies liées à l'hème, c'est-à-dire la maladie impliquant un trouble de l'hème au moins en tant que symptôme. Les maladies liées à l'hème englobent les maladies liées à l'hémoglobine ou les maladies RBC.

Le cas échéant, les applications sont des applications in vitro.

*Procédé de prédiction*

Pour cette application, il est proposé un procédé permettant de prédire qu'un sujet est à risque de souffrir d'une maladie liée à l'hème ou à une hémoprotéine.

Le procédé de prédiction comprend une étape pour mettre en oeuvre les étapes du procédé pour déterminer au moins une teneur en protéine dans un échantillon biologique du sujet, afin d'obtenir des paramètres déterminés.

Le procédé de prédiction comprend également une étape consistant à prédire que le sujet est à risque de souffrir de la maladie liée à l'hème ou à une hémoprotéine sur la base des paramètres déterminés.

*Procédé de diagnostic*

Cette application correspond à un procédé de diagnostic d'une maladie liée à l'hème ou à une hémoprotéine.

Le procédé de diagnostic comprend la mise en oeuvre des étapes du procédé de détermination d'au moins une teneur en protéines dans un échantillon biologique du sujet, afin d'obtenir des teneurs déterminées en protéines.

Le procédé de diagnostic comprend également la réalisation d'une étape de diagnostic d'une maladie liée à l'hème ou à une hémoprotéine sur la base de la teneur déterminée en protéine.

*Procédé de traitement*

Cette application correspond à une méthode de traitement d'une maladie liée à l'hème ou à une hémoprotéine (suivi).

Le procédé de traitement comprend la mise en oeuvre des étapes du procédé de détermination d'au moins une teneur en protéines dans un échantillon biologique du sujet, afin d'obtenir des teneurs déterminées en protéines.

Le procédé de traitement comprend également l'administration d'un médicament (transplantation de moelle osseuse, thérapie génique) traitant la maladie liée à l'hème ou à une hémoprotéine sur la base de la teneur déterminée en protéines.

*Procédé de définition des stades*

Dans cette application, il est proposé une méthode pour définir les étapes d'une maladie liée à l'hème ou à une hémoprotéine.

Les stades d'une maladie sont les différents niveaux d'une maladie. La définition des stades est généralement définie en fonction des symptômes de la maladie. Le procédé de définition comprend la mise en oeuvre des étapes d'un procédé de détermination d'au moins une teneur en protéines dans un échantillon biologique du sujet, afin d'obtenir des teneurs déterminées en protéines.

Le procédé de définition comprend ensuite la définition des étapes de la maladie liées à l'hème ou à une hémoprotéine sur la base des teneurs déterminées en protéines.

*Procédé d'identification d'une cible*

Cette application correspond à un procédé d'identification d'une cible thérapeutique pour la prévention et/ou le traitement d'une maladie liée à l'hème ou à une hémoprotéine.

Le procédé d'identification comprend la mise en oeuvre des étapes du procédé de détermination d'au moins une teneur en protéine dans un échantillon biologique d'images d'un premier sujet, le premier sujet étant un sujet souffrant de la maladie liée à l'hème ou à une hémoprotéine.

Le procédé d'identification comprend également la mise en oeuvre des étapes du procédé de détermination d'au moins une teneur en protéine dans un échantillon biologique d'un deuxième sujet, afin d'obtenir une deuxième teneur déterminée en protéine, le deuxième sujet étant un sujet ne souffrant pas de maladie liée à l'hème ou à une hémoprotéine.

Le procédé d'identification comprend en outre une étape de sélection d'une cible thérapeutique basée sur la comparaison de la première teneur déterminée en protéine et du deuxième teneur déterminée en protéine.

*Procédé d'identification d'un biomarqueur*

Dans cette application, un procédé d'identification d'un biomarqueur est proposé.

Le biomarqueur peut être un biomarqueur parmi un biomarqueur diagnostique d'une maladie liée à l'hème ou à une hémoprotéine, un biomarqueur de susceptibilité d'une maladie liée à l'hème ou à une hémoprotéine, un biomarqueur pronostique d'une maladie liée à l'hème ou à une hémoprotéine ou un biomarqueur prédictif en réponse au traitement d'une maladie liée à l'hème ou à une hémoprotéine.

Le procédé d'identification comprend la mise en oeuvre des étapes du procédé de détermination d'au moins une teneur en protéine dans un échantillon biologique d'un premier sujet, afin d'obtenir une première teneur déterminée, le premier sujet étant un sujet souffrant de la maladie liée à l'hème ou à une hémoprotéine.

Le procédé d'identification comprend la mise en oeuvre des étapes du procédé de détermination d'au moins une première teneur déterminée en protéine dans un échantillon biologique d'images d'un deuxième sujet, afin d'obtenir une deuxième teneur déterminée en protéine, le deuxième sujet étant un sujet ne souffrant pas de maladie liée à l'hème ou à une hémoprotéine.

Le procédé d'identification comprend la sélection d'un biomarqueur sur la base de la comparaison de la première teneur déterminée en protéine et de la deuxième teneur déterminée en protéine.

*Procédé de criblage d'un composé*

Cette application correspond à un procédé de criblage d'un composé.

Le composé est un médicament.

Le médicament a un effet sur une cible thérapeutique connue, pour prévenir et / ou traiter une maladie liée à l'hème ou à une hémoprotéine.

Le procédé de criblage comprend la mise en oeuvre des étapes du procédé de détermination d'au moins une teneur en protéine dans un échantillon biologique d'un premier sujet, afin d'obtenir une première teneur déterminée en protéine, le premier sujet étant un sujet souffrant de la maladie liée à l'hème ou à une hémoprotéine et ayant reçu le composé. Dans ce contexte, le terme «recevoir» englobe tous les modes d'administration du médicament.

Le procédé de criblage comprend également la mise en oeuvre des étapes du procédé de détermination d'au moins une teneur en protéine dans un échantillon biologique d'un deuxième sujet, afin d'obtenir une deuxième teneur déterminée en protéine, le deuxième sujet étant un sujet souffrant de la maladie liée à l'hème ou à une hémoprotéine et n'a pas reçu le composé.

Le procédé de criblage comprend en outre la sélection d'un composé sur la base de la comparaison de la première teneur déterminée en protéine et de la deuxième teneur déterminée en protéine.

*Procédé de qualification ou de disqualification*

Cette application correspond à une méthode de qualification et de disqualification des poches de sang.

Les sacs sont des conteneurs.

Les sacs contiennent un échantillon biologique de sujet.

Le procédé de qualification ou de disqualification comprend la mise en oeuvre des étapes du procédé de détermination d'au moins une teneur en protéines dans la poche médicale, afin d'obtenir des teneurs déterminées en protéines.

Le procédé de qualification ou de disqualification comprend également la qualification ou la disqualification de poches médicales basées sur les teneurs déterminées en protéines. Par exemple, dans le cas où le contenu en protéines montre que la qualité de l'échantillon est tellement dégradée que l'échantillon ne peut plus être utilisé, le sac est disqualifié. L'idée d'une telle application spécifique est d'analyser le contenu de l'hémolyse dans le milieu de conservation, mais également s'il existe un doute de collecter des globules rouges pour incubation dans un PBS isotonique à pH 7,4 (le pH des poches diminue) avec le temps de conservation et se situe généralement entre 6 et 7) en présence d'albumine 100-500 $\mu$M (in vivo) pour récupérer tout ce qui pourrait interagir avec la membrane et également pour mesurer le composant le plus fragile pouvant réduire le rendement transfusionnel. D'où cette application dans le cadre d'un contrôle de qualité.

*Procédé de suivi d'un traitement*

Cette application correspond à un procédé de suivi d'un traitement contre la maladie liée à l'hème ou à une hémoprotéine chez un sujet souffrant de la maladie liée à l'hémoglobine et ayant reçu le traitement.

Le procédé de suivi comprend la mise en oeuvre des étapes du procédé de détermination d'au moins une teneur en protéines dans un échantillon biologique du sujet, afin d'obtenir des teneurs déterminées en protéines.

Le procédé de suivi comprend en outre la surveillance des teneurs déterminées en protéines avec la surveillance d'un traitement contre une maladie liée à l'hème ou à une hémoprotéine chez un sujet souffrant de la maladie liée à l'hème ou à une hémoprotéine et ayant reçu le traitement.

De telles applications correspondent à de nombreuses applications possibles parmi lesquelles certaines seront décrites dans la suite.

Le procédé peut notamment être utilisé dans:

- la surveillance du traitement des porphyres à l'hème arginate;
- la surveillance d'un traitement à l'hémopexine purifiée ou recombinante;
- surveillance de l'ajout d'un substitut sanguin dérivé de l'Hb;
- surveillance des traitements induisant une hémolyse tels que les ecmo (circulation extra-corporelle, hémodialyse ou prothèses cardiaques);
- caractérisation des échanges plasmatiques, de la plasmaphérèse (clairance de l'hémoglobine et de l'hème après addition de leurs capteurs naturels l'haptoglobine et de l'hémopexine), calcul du rendement d'échange (dilution du plasma du patient), et
- utiliser la concentration d'hème non liée à l'hémopexine pouvant être impliquée dans des réactions redox liées à l'HSA ou susceptibles de diffuser vers les tissus et de modifier l'homéostasie tissulaire.

En conclusion, il a été proposé une méthode pour déterminer au moins une protéine dans le sang qui soit plus précise tout en restant simple à mettre en oeuvre. Cela permet de prendre en compte de nombreuses applications pour les maladies liées à l'hème et l'Hb.

Conclusions :

Les caractéristiques spectrales des différentes espèces à doser sont donc uniques. En utilisant une réaction biochimique d'une espèce afin d'induire un changement spectral spécifique pour son dosage et/ou en utilisant une approche mathématique séquentielle la plus appropriée pour doser chaque espèce à partir d'un spectre du plasma ou du sérum il est donc possible de mesurer in vitro le statut des biomarqueurs de l'hémolyse *in vivo* plus particulièrement de la composante intravasculaire et/ou de la dysérythropoïèse. De manière plus générale ces biomarqueurs seront liés à une pathologie associée à un défaut de l'anabolisme et/ou du catabolisme de l'hème et de l'Hb.

Légendes des figures

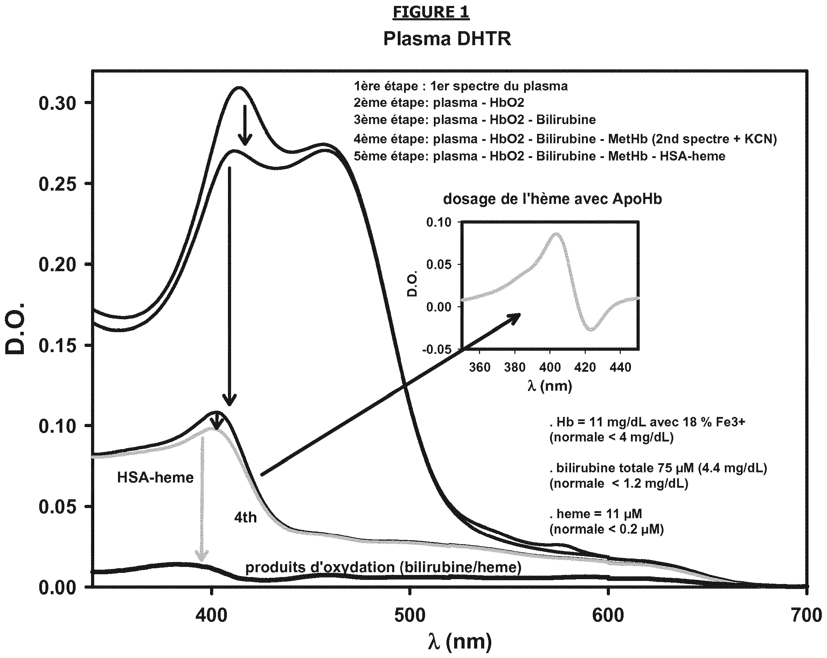

Figure 1: Méthode de dosage de l'$HbO_2$, metHb, de l'hème plasmatique et des produits de dégradation et d'oxydation de l'hème par une approche spectrophotométrique UV/visible sur un échantillon de plasma d'un patient atteint de drépanocytose après hémolyse iatrogène post-transfusionnelle (DHTR).

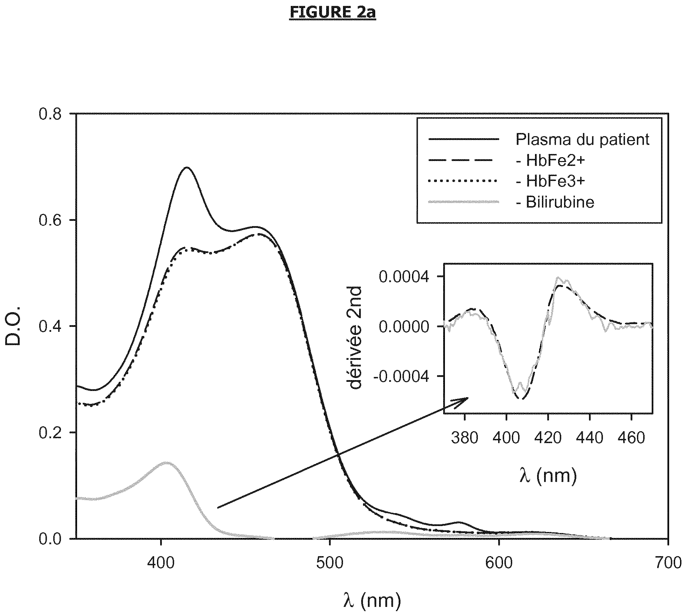

Figure 2a: Méthode de dosage de l'hémoglobine $Fe^{2+}O_2$ et $Fe^{3+}$, de l'hème plasmatique et des produits de dégradation et d'oxydation de l'hème par une approche spectrophotométrique UV/visible sur un échantillon de plasma d'un patient atteint de drépanocytose avec un problème mécanique de valve cardiaque

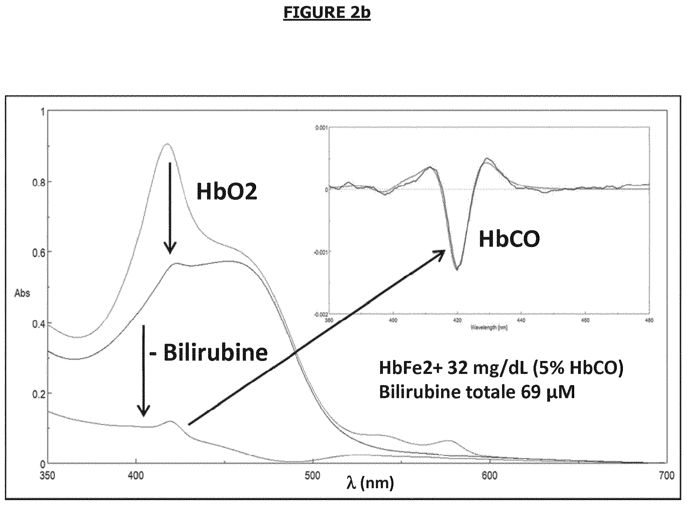

Figure 2b: Méthode de dosage de l'hémoglobine $Fe^{2+}O_2$, $Fe^{3+}CO$ et $Fe^{3+}$ (non détectable), de l'hème plasmatique et des produits de dégradation et d'oxydation de l'hème par une approche spectrophotométrique UV/visible sur un échantillon de plasma d'un patient atteint de drépanocytose présentant une hémolyse modérée sous hydroxyurée.

Figure 2 c et 2d: Méthode de dosage de l'hémoglobine $Fe^{2+}O_2$ et $Fe^{3+}CO$ et Fe3+, de l'hème plasmatique et des produits de dégradation et d'oxydation de l'hème par une approche spectrophotométrique UV/visible sur un échantillon de plasma d'un patient atteint de drépanocytose présentant une hémolyse modérée associée à des facteurs de risques aggravants : taux élevé de globules rouges denses, déficit en G6PD et multitraitements pharmaceutiques.

Figures 3a et 3b: Méthode de dosage de la bilirubine plasmatique totale par spectrophotométrie UV/visible.

Figure 4: Dosage de l'hémoglobine $Fe^{3+}$ plasmatique par spectrophotométrie UV/visible utilisant du KCN.

Figures 5a, 5b, 5c, 5d et 5e : Spectres standards de l'$HbO_2$, l'hème-HSA, la bilirubine, la metHb +/- KCN, l'hème-Hx et leurs dérivées secondes associées.

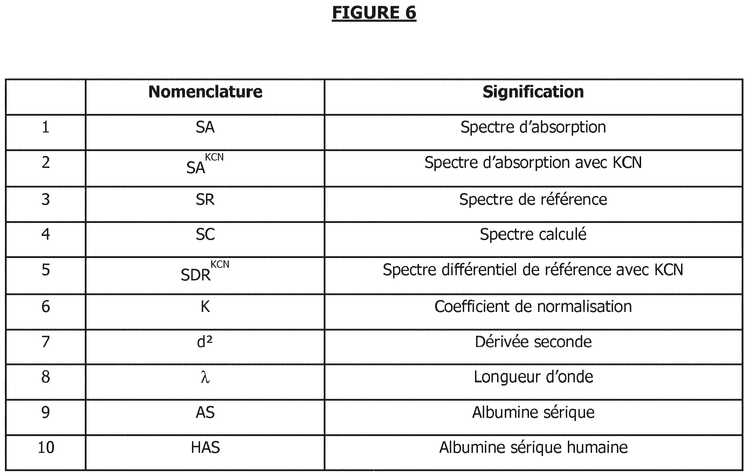

Figure 6 : abréviations utilisées dans la demande de brevet

Exemples

Exemple 1 (Figure 1)

Spectre d'absorption UV/visible d'un patient DHTR. Les différentes espèces liées à l'hème ont été mesurées par une approche mathématique basée sur des mesures spectrales expérimentales couplées aux spectres des formes pures de chaque espèce considérée.

Matériels et Méthodes : plasma dilué au 1/6$^{ème}$ (trajet optique 4 mm) dans un PBS 50 mM phosphate de potassium 50 mM NaCl pH 7,4. Ajout de KCN 0,2 mM à partir d'une solution mère à 50 mM. Soit 2,5 $\mu$l dans 600 $\mu$l total de plasma dilué (facteur de dilution 1,004).

premier spectre d'absorption SA: spectre mesuré du plasma

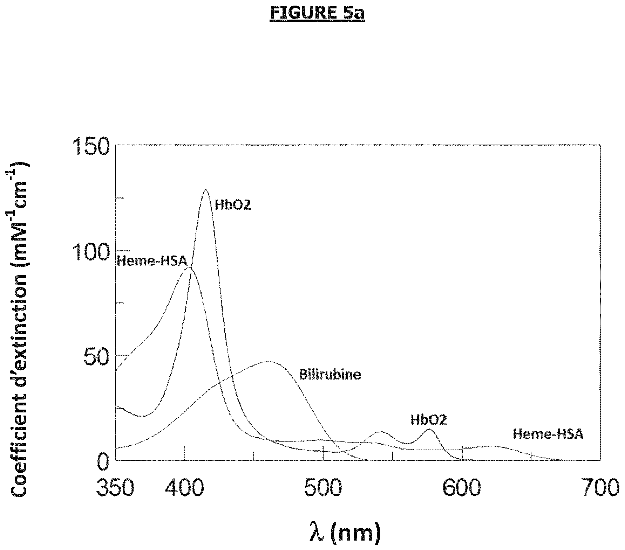

deuxième spectre calculé intermédiaire : la composante HbO$_2$ a été enlevée par soustraction d'un spectre d'hémolysat complètement oxygéné sous air (voir Figure 5a) basée sur la dérivée seconde entre 500 and 600 nm (typiquement, le pic à 577 nm présente une bande intense négative après dérivée seconde). En effet, les pics étroits dans ce domaine d'absorption proviennent seulement du spectre HbO$_2$ alors que les autres composants spectraux majeurs ici présents (bilirubine, metHb (HbFe$^{3+}$) et hème-HAS) ont des dérivées secondes moins intenses du fait de leurs larges pics. HAS= albumine sérique humaine.

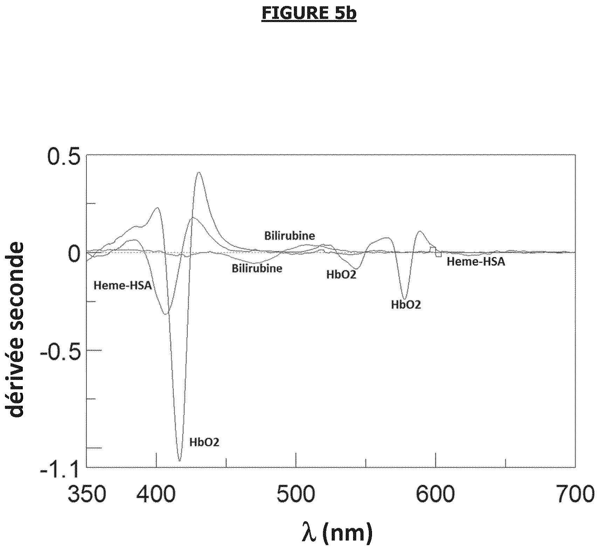

troisième spectre calculé intermédiaire : la composante bilirubine a été enlevée après soustraction du spectre de bilirubine liée à l'HAS (voir Figure 5a) basée sur la dérivée seconde entre 440 nm et 520 nm (Figure 5b).

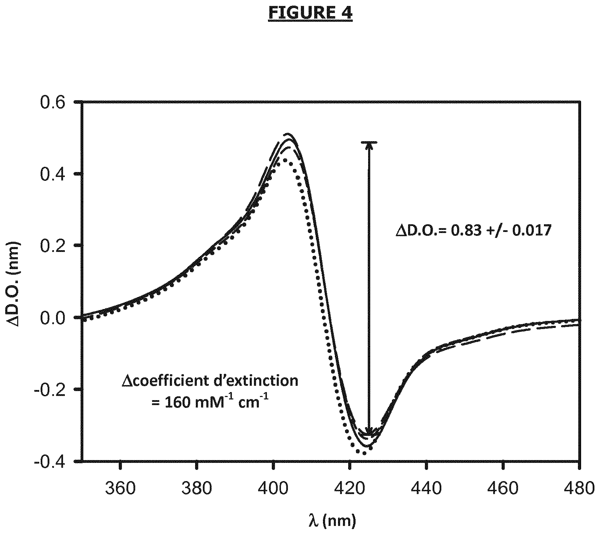

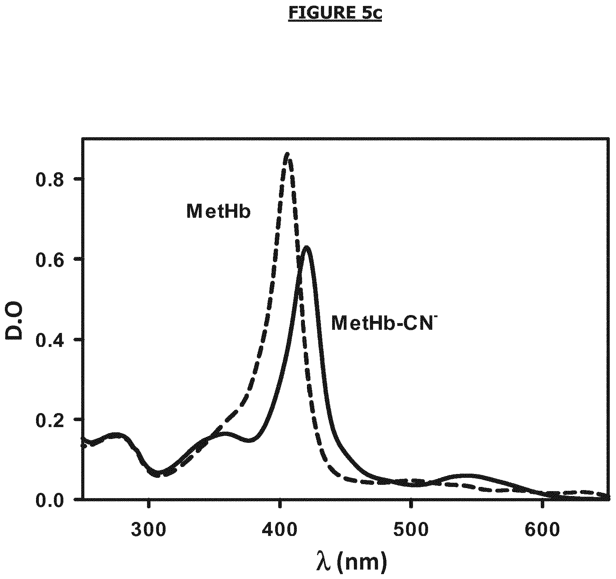

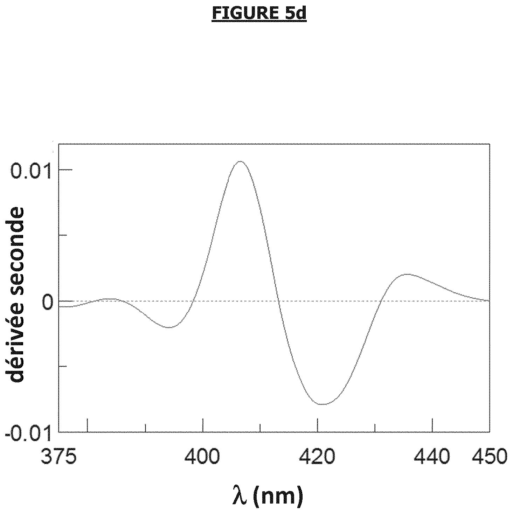

quatrième spectre calculé intermédiaire : après enregistrement d'un deuxième spectre du même plasma après ajout de KCN pour induire un changement d'absorption spécifique de cette espèce après liaison au CN du fer ferrique, la composante méthémoglobine (metHb) a été soustraite une fois son dosage effectué (voir Figures 4, 5a, 5b, 5c et 5d). Il est à noter qu'en cas de forte contribution de la composante metHb sa soustraction a été faite avant celle de la bilirubine voire avant celle de l'HbO$_2$. A ce stade, le spectre résiduel était majoritairement dû à ce qui est appelé de l'hème libre qui est en fait de la metalbumine ou de l'hème-HAS pour des plasmas de patients avec une hémolyse chronique ou une forte hémolyse.

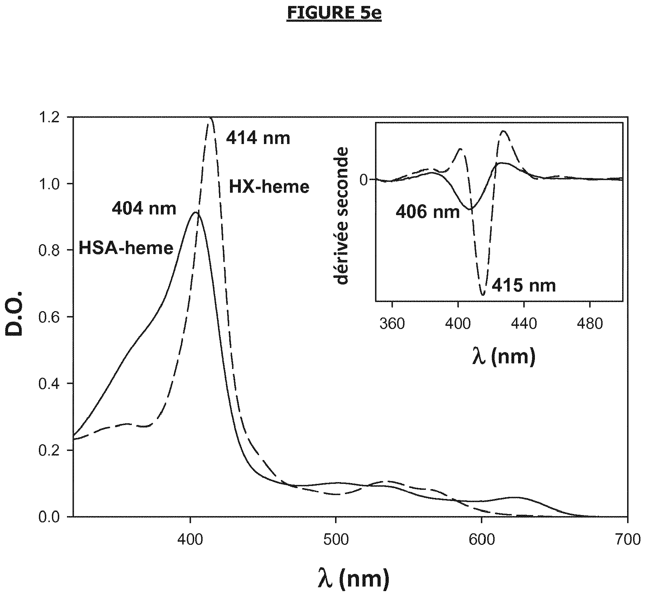

Cependant, l'hème-hémopexine (Hème-Hx) peut être présente mais toujours en quantité plus faible du fait du rapport de concentration entre HAS et hémopexine $\geq$ 40 dans les conditions normales qui est augmenté du fait de l'hémolyse par la clairance rapide du complexe hème-hémopexine par endocytose hépatique jusqu'à sa déplétion du plasma comme dans les exemples présentés. Le calcul de la concentration d'hème-HAS a été fait à partir de la dérivée seconde du spectre pris entre 390 nm et 440 nm (typiquement le pic à 403-404 nm donne une bande négative de dérivée seconde décalée à 406 nm, voir Figure 5e). Il est à noter que la dérivée seconde de l'hème-hémopexine est plus intense que l'hème-HAS (Figure 5e). La présence de 10-20 % d'hème-hémopexine a pour conséquence un déplacement notable du minimum de la dérivée seconde mesurée vers celui de l'hème-hémopexine. Dans ce cas de figure la dérivée seconde mesurée peut être simulée comme une combinaison linéaire des deux complexes protéiques majoritaires *in vivo* pour la liaison de l'hème plasmatique.

Cinquième spectre calculé intermédiaire: le spectre d'absorption résiduelle calculé après soustraction de la contribution spectrale de l'hème-HAS (déplétion en hémopexine). Cette analyse spectroscopique n'est pas restreinte aux espèces liées à l'hème listées plus haut ; à titre d'exemple la myoglobine dans le cas de myolyse peut être mesurée.

Résultats : Les valeurs de l'hémolyse sont listées dans la figure : pour l'HbO$_2$, la MetHb, la bilirubine totale, l'hème plasmatique lié majoritairement à l'HAS. Etant donné que le prélèvement est effectué à distance du pic de l'hémolyse (+24h) le dosage de l'hème est probablement le reflet du statut de l'hémolyse juste après la transfusion et avant l'hémolyse massive qui habituellement est suivie d'une très forte hémoglobinurie. En effet l'absence de liaison de l'hème à l'hémopexine indique de facto sa quasi-déplétion du compartiment vasculaire. La clairance de l'hème est ainsi très ralentie.

Conclusions : La mesure de l'hème plasmatique soulève la question du rôle de l'hémolyse chez le drépanocytaire majorée par l'hémolyse après transfusion des concentrés de GRs, dont une population est fragilisée après le stockage, sur l'hémolyse majeure post-transfusionnelle. En effet dans certaines DHTR on note l'absence d'anticorps détectables contre les GRs du donneur. L'hémolyse avant DHTR pourrait par exemple induire une activation de la voie alterne du complément.

$$[\text{Hb plasmatique libre}] = [\text{Hb totale, en hème}] - [\text{Haptoglobine, g/L}]/85000 \times 4$$

Le coefficient d'extinction à 577 nm pour le spectre d'absorption HbO$_2$ est de 15,5 /mM/cm.

Le ratio (metHb (GbFe$^{3+}$) + hème (Fe$^{3+}$) plasmatique)/hème total peut être utilisé comme index d'un stress oxydant dans le plasma.

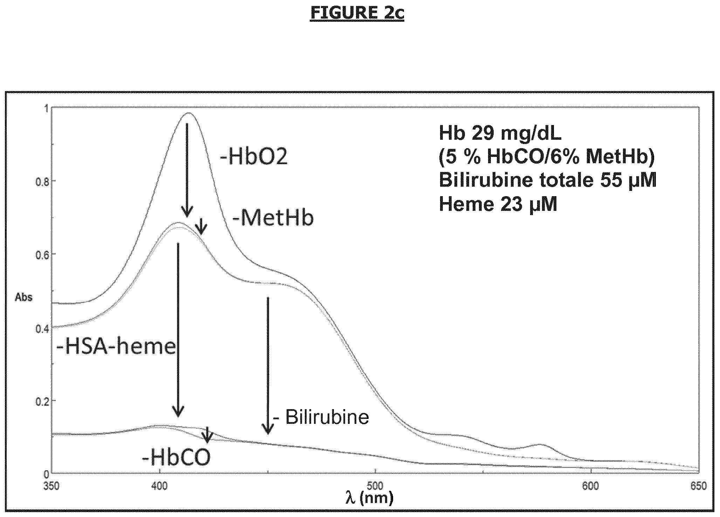

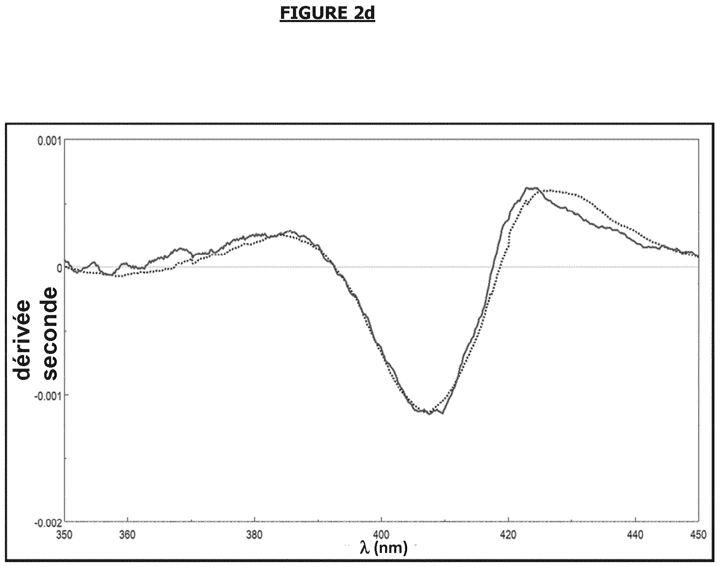

Exemple 2 (Figures 2 a,b,c,d) : dosage sur un échantillon de patient drépanocytaire avec un problème mécanique de valve cardiaque

Après soustraction des différentes composantes spectrales en utilisant les dérivées secondes pour $HbFe^{2+}(O_2)$, la bilirubine-HAS et le dosage de $HbFe^{3+}$ après ajout de KCN, la présence d'hème plasmatique a été révélée dans le violet ici chez un patient drépanocytaire avec un problème mécanique de valve cardiaque (Figure 2a).

Matériels et Méthodes : plasma dilué au 1/6ème (trajet optique 4 mm) dans un PBS 50 mM phosphate de potassium 50 mM NaCl pH 7.4. Ajout de KCN 0.2 mM à partir d'une solution mère à 50 mM. Soit 2.5 $\mu$l dans 600 $\mu$l total de plasma dilué (facteur de dilution 1.004).

Résultats : suivant la même méthodologie que la Figure 1 les valeurs des biomarqueurs de l'hémolyse sont : Hb plasmatique 13 $\mu$M (5 % $Fe^{3+}$), Bilirubine totale 130 $\mu$M, Hème plasmatique 15 $\mu$M. De même l'absence de liaison de l'hème à l'hémopexine indique une quasi-déplétion de ce « scavenger » de l'hème.

Dans l'insert est montrée la dérivée seconde de la forme spectrale hors globine (trait en pointillé) et qui correspond bien à la présence d'hème plasmatique liée à l'HAS (trait plein).

Conclusions : Le dosage des biomarqueurs de l'hémolyse peut être utilisé pour diagnostiquer et quantifier l'impact de cette hémolyse mécanique. D'autres hémolyses de ce type peuvent faire l'objet d'un suivi de l'hémolyse comme dans le cas de CEC (chirurgie cardiaque par ex), coeur artificiel.

Dosage sur un échantillon de patient atteint de drépanocytose présentant une hémolyse modérée sous HU (Figure 2b)

Matériels et Méthodes : plasma dilué au 1/6ème (trajet optique 1 cm) dans un PBS 50 mM phosphate de potassium 50 mM NaCl pH 7.4. Ajout de KCN 0.2 mM à partir d'une solution mère à 50 mM. Soit 2,5 $\mu$l dans 600 $\mu$l total de plasma dilué (facteur de dilution 1,004).

Résultats : suivant la même méthodologie que la Figure 1 les valeurs des biomarqueurs de l'hémolyse sont données dans la figure. Après soustraction des différentes composantes spectrales en utilisant les dérivées secondes pour $HbFe^{2+}(O_2)$, la bilirubine-HAS et le dosage de $HbFe^{3+}$ après ajout de KCN (non présente ; plasma hème < 1 $\mu$M), la présence d'HbCO a été révélée dans le violet ici chez un patient drépanocytaire sous hydroxyurée avec une hémolyse modérée mais cependant 10 fois supérieure à la moyenne des patients drépanocytaires avec une faible hémolyse intra-vasculaire (Figure 2b). Dans l'insert est montrée la dérivée seconde de la composante spectrale dans le violet après soustraction de l'$HbO_2$ et la bilirubine liée à l'HAS en comparaison avec la référence de l'HbCO.

Conclusions : l'absence d'hème plasmatique pourrait résulter d'une forte activité hème-oxygénase révélée par la présence d'HbCO ou d'un effet du traitement à l'hydroxyurée (diminution de la falciformation et de la déshydratation des GRs).

Dosage sur un échantillon de patient atteint de drépanocytose présentant une hémolyse modérée avec un facteur de risque aggravant.

Matériels et Méthodes : plasma dilué au 1/6ème (trajet optique 1 cm) dans un PBS 50 mM phosphate de potassium 50 mM NaCl pH de 7,4. Ajout de KCN 0,2 mM à partir d'une solution mère à 50 mM. Soit 2,5 $\mu$l dans 600 $\mu$l total de plasma dilué (facteur de dilution 1,004).

Résultats : suivant la même méthodologie que la Figure 1 les valeurs des biomarqueurs de l'hémolyse sont données dans la Figure 1. Après soustraction des différentes composantes spectrales en utilisant les dérivées secondes pour $HbFe^{2+}(O_2)$ et la bilirubine, le dosage de la $metHbFe^{3+}$ après ajout de KCN, l'HbCO (Figure 2c), l'hème plasmatique lié à l'HAS (Figures 2c et 2d) a été révélé dans le violet ici chez un patient drépanocytaire avec une hémolyse modérée. La présence d'hème plasmatique chez ce patient avec une hémolyse modérée est cependant 10 fois supérieure à la moyenne des patients drépanocytaires sans hémolyse intra-vasculaire.

Conclusions : Chez ce patient cette quantité élevée d'hème plasmatique peut être due à plusieurs facteurs qui sont à considérer:

1) la présence de 30% de globules rouges denses plus hémolytiques et sujet à la perte de matériel membranaire via la formation de microparticules,

2) la présence d'un déficit enzymatique en G6PD qui est responsable d'un stress oxydant dans le GR, et

3) l'accumulation de différents traitements médicamenteux (cette analyse de l'hémolyse peut être utilisée dans le cadre d'un suivi longitudinal du traitement).

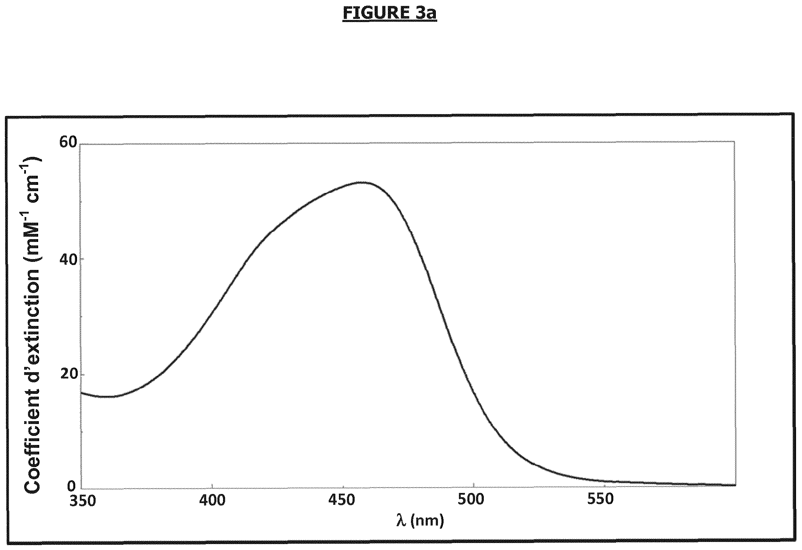

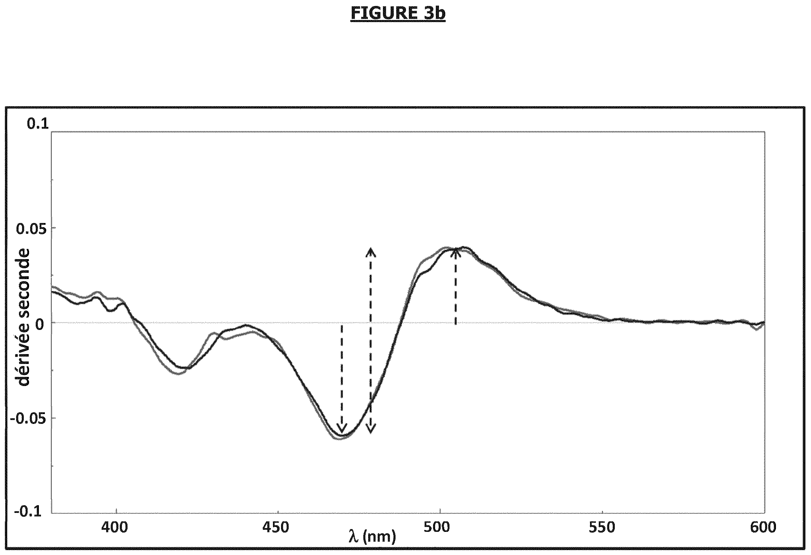

Exemple 3 (Figures 3 a,b) : dosage de la bilirubine plasmatique totale

Matériels et Méthodes : plasma d'un patient avec une faible hémolyse intravasculaire (stomatocytose héréditaire avec déshydratation) dilué au 1/6$^{ème}$ (trajet optique 1 cm) dans un PBS 50 mM phosphate de potassium 50 mM NaCl pH 7.4. La faible contribution spectrale de l'Hb plasmatique a été soustraite comme décrit Figure 1. Après dissolution de l'HAS humaine lyophilisée « fatty free » dans ce même tampon puis ajout de bilirubine (< HAS) tel que décrit dans l'exemple 1 (produits Sigma) un spectre de hème-HAS de référence est enregistré et normalisé.

Résultats : Le spectre du plasma après soustraction de l'HbO$_2$ est montré en Figure 3a. Il correspond à la forme spectrale de la bilirubine (non conjuguée et plus faiblement conjuguée) liée l'HAS. Ce spectre est similaire à celui obtenu à partir des spectres de référence de la bilirubine liée à l'HAS humaine (produits commerciaux). En effet les dérivées 2$^{nd}$ sont quasi-identiques (Figure 3b).

Conclusions : Le maximum à 501 nm de préférence et le minimum à 470 nm ou la différence entre les deux de la dérivée seconde d'un plasma peuvent être analysés pour estimer la concentration en bilirubine totale plasmatique. Si l'ensemble des contributions spectrales liées à l'hème a été soustrait l'analyse peut également reposée sur une simple mesure de DO au maximum d'absorbance en utilisant le coefficient d'extinction de la bilirubine-HAS.

Exemple 4 (Figure 5 a,b,c,d,e)

Matériels et Méthodes : Les spectres d'absorption UV/visible des principales espèces spectrales mesurées dans le plasma d'un patient avec une anémie hémolytique dont une composante intravasculaire est mesurée dans un tampon phosphate 50 mM NaCl de pH 7,4, 25 °C.

Résultats : Les_spectres standards normalisés de l'HbO$_2$, l'hème-HAS et la bilirubine liée à l'HAS (coefficient d'extinctions molaires mM$^{-1}$cm$^{-1}$, Figure 5a) et leur dérivé seconde associée (Figure 5b) montrent les caractéristiques suivantes :

HbO$_2$ = 15 /mM/cm à 577 nm (Dans la dérivée seconde a montré 2 maxima dans le violet à 400 nm et 430 nm et un minimum à 416 nm dans le vert et le rouge également deux minima à 543 nm et 577 nm.

Hème-HAS = 92 /mM/cm à 403 nm - 404 nm (pour la dérivée seconde deux maxima à 385 nm et 426 nm et un minimum à 406 nm - 407 nm. Dans le rouge un minimum est observé vers 625 nm absent de toutes autre formes spectrales majoritaires et dont il est possible de tirer avantage pour la mesure de forte concentration d'hème-HAS dans l'échantillon et/ou en diminuant la dilution du plasma au 1/2).

bilirubine-HAS = 47 mM$^{-1}$cm$^{-1}$ à 459 +/- 1 nm (pour la dérivée seconde le minimum a été déplacé vers 470 nm et le maximum vers 507 nm).

Les spectres UV/visible pour la metHb (ligne pointillée) et la metHb-CN (ligne pleine) sont présentés Figure 5c. Les conditions expérimentales était 50 mM potassium phosphate 20 mM NaCl pH 7.4, 25 °C. L'absorption différentielle dans la bande de Soret est montrée dans la Figure 4. La Figure 5d montre la dérivée seconde de ce spectre différentiel avec un maximum à 406 nm et un minimum à 421 nm pour la dérivée seconde à comparer à 404 nm /424 nm pour le spectre différentiel +/- KCN montré en Figure 4.

Enfin, la Figure 5e montre la comparaison des spectres UV/visible des deux protéines sériques majoritaires pour la liaison de l'hème : HAS et Hx (concentration 10 μM pour chacune). En insert est montrée leur dérivée seconde. La dérivée seconde de Hx est plus intense que celle de HAS. Ainsi une faible fraction d'hème liée à l'Hx vs l'HAS aura pour conséquence un déplacement de la dérivée seconde de 406 nm vers 415 nm. Par exemple pour 10 % hème-Hx le minimum est déplacé de 406 nm -> 411 nm et dès 25 % de 406 nm - > 414 nm. Cela montre confirme la quasi-déplétion en Hx dans les plasmas de patients avec une hémolyse pathologique présentés dans les exemples plus haut.

**Revendications**

1. Procédé de détermination d'une teneur d'au moins une protéine présente dans un fluide biologique comprenant les étapes de :

   a) obtenir un spectre d'absorption SA d'un échantillon du fluide biologique ;
   b) soustraire audit spectre d'absorption SA une composante spectrale associée à l'oxyhémoglobine afin d'obtenir un spectre calculé intermédiaire SC1 ; ladite composante spectrale associée à l'oxyhémoglobine étant obtenue par normalisation d'un spectre de référence SR1 d'oxyhémoglobine, ladite normalisation étant effectuée par rapport audit spectre d'absorption SA en calculant un coefficient de normalisation K1 à une longueur d'onde choisie dans le groupe constitué de 416 nanomètres, 543 nanomètres et 577 nanomètres ;
   c) soustraire audit spectre calculé intermédiaire SC1 une composante spectrale associée à la méthémoglobine afin d'obtenir un spectre calculé intermédiaire SC2 ; ladite composante spectrale associée à la méthémoglobine étant obtenue à partir d'au moins un spectre choisi parmi le spectre d'absorption SA, le spectre d'absorption de l'échantillon en présence d'un ajout de KCN, le spectre de référence de la méthémoglobine (SR2) et le spectre de référence de la méthémoglobine (SR2) en présence de KCN ;

d) soustraire audit spectre calculé intermédiaire SC2 une composante spectrale associée à la bilirubine afin d'obtenir un spectre calculé intermédiaire SC3 ; ladite composante spectrale associée à la bilirubine étant obtenue par normalisation d'un spectre de référence SR3 de bilirubine, ladite normalisation étant effectuée par rapport audit spectre calculé intermédiaire SC2 en calculant un coefficient de normalisation K3 dans un domaine de longueurs d'ondes compris entre 200 nanomètres et 800 nanomètres, de préférence compris entre 350 nanomètres et 700 nanomètres ;

e) soustraire audit spectre calculé intermédiaire SC3 une composante spectrale associée à l'hème lié à l'albumine sérique (hème-AS) afin d'obtenir un spectre calculé intermédiaire SC4 ; ladite composante spectrale associée à l'hème -AS étant obtenue par normalisation d'un spectre référence SR4 de l'hème--AS, ladite normalisation étant effectuée par rapport audit spectre calculé intermédiaire SC3 en calculant un coefficient de normalisation K4 à une longueur dans un domaine de longueurs d'ondes compris entre 200 nanomètres et 800 nanomètres, de préférence compris entre 300 nanomètres et 700 nanomètres, de préférence compris entre 380 nanomètres et 450 nanomètres; et éventuellement

f) soustraire audit spectre calculé intermédiaire SC4 une composante spectrale associée à au moins une autre protéine héminique présente dans le fluide biologique afin d'obtenir un spectre calculé intermédiaire SC5 ; ladite composante spectrale associée à ladite au moins une autre protéine héminique étant obtenue par normalisation d'un spectre de référence SR5 de ladite au moins une autre protéine héminique ; ladite normalisation étant effectuée par rapport audit spectre calculé intermédiaire SC5 en calculant un coefficient de normalisation K5 dans un domaine de longueurs d'ondes compris entre 200 nanomètres et 700 nanomètres.

2. Procédé selon la revendication **1,** dans lequel le spectre de l'étape a) est obtenu dans un domaine de longueurs d'ondes comprenant tout ou partie de l'ultra-violet et tout ou partie du visible, de préférence dans un domaine de longueurs d'ondes allant de 200 nanomètres à 800 nanomètres, de préférence allant de 300 nanomètres à 700 nanomètres.

3. Procédé selon la revendication **1** ou **2,** dans lequel le spectre de l'étape a) est obtenu à partir d'un échantillon du fluide biologique dilué, afin d'obtenir une densité optique maximale du spectre d'absorption SA inférieure ou égale à 2, de préférence inférieure ou égale à 1.

4. Procédé selon l'une quelconque des revendications **1** à **3,** dans lequel l'échantillon du fluide biologique est tamponné à un pH inférieur à 8, de préférence à un pH compris entre 7,35 et 7,45, de préférence égal à 7,4.

5. Procédé selon l'une quelconque des revendications **1** à **4,** dans lequel le coefficient de normalisation K1 est calculé par détermination du rapport entre les valeurs des dérivées secondes du spectre d'absorption et du spectre de référence SR1 à l'une des longueurs d'onde du groupe.

6. Procédé selon l'une quelconque des revendications **1** à **5,** dans lequel le coefficient de normalisation K1 est calculé à une longueur d'onde de 577 nanomètres.

7. Procédé selon l'une quelconque des revendications **1** à **6,** dans lequel l'oxyhémoglobine est au moins l'une de l'oxyhémoglobine A et de l'oxyhémoglobine F.

8. Procédé selon l'une quelconque des revendications **1** à **7,** dans lequel l'étape c) est réalisée en soustrayant la composante spectrale associée à la méthémoglobine, obtenue par normalisation d'un spectre de référence SR2 de la méthémoglobine, ladite normalisation étant effectuée par rapport audit spectre calculé intermédiaire SC1 en calculant un coefficient de normalisation K2 à une longueur d'onde comprise entre 350 nanomètres et 700 nanomètres, de préférence comprise entre 380 nanomètres et 440 nanomètres.

9. Procédé selon l'une quelconque des revendication **1** à **8,** dans lequel le coefficient de normalisation K3 est calculé par détermination du rapport entre les valeurs des dérivées secondes du spectre d'absorption SC2 et du spectre de référence SR3 à une longueur d'onde étant entre 350 nanomètres et 700 nanomètres.

10. Procédé selon l'une quelconque des revendications **1** à **9,** dans lequel le coefficient de normalisation K4 est calculé par détermination du rapport entre les valeurs des dérivées secondes du spectre d'absorption et du spectre de référence SR4 à une longueur d'onde allant d'environ 350 nm à environ 460 nm.

11. Procédé selon l'une quelconque des revendications **1** à **10,** dans lequel ladite au moins une autre protéine héminique présente dans le fluide biologique est choisie parmi l'hème lié à l'hémopexine, l'hémoglobine carboxylée, un biomarqueur du catabolisme de l'hème par l'hème oxygénase, la myoglobine, les porphyrines, les produits de dégradation de la bilirubine, la porphobiline des urines, la stercobiline et l'urobiline.

12. Procédé selon l'une quelconque des revendications **1** à **11,** dans lequel ladite au moins une autre protéine présente dans le fluide biologique est l'hème lié à l'hémopexine (hème-Hx), et dans lequel les étapes e) et f) sont combinés en l'étape e') étant réalisée en soustrayant la composante spectrale associée à la combinaison linéaire de l'hème-AS et l'hème-Hx, ladite composante spectrale étant obtenue par normalisation d'un spectre de référence SR4' de la contribution de deux spectres de référence SR4 de l'hème-AS et SR5 de l'hème-Hx, ladite normalisation étant effectuée par rapport audit spectre calculé intermédiaire SC3 en calculant deux coefficients de normalisation K4 et K5 à une longueur d'onde comprise dans un domaine de longueurs d'ondes compris entre 200 nanomètres et 800 nanomètres, de préférence compris entre 300 nanomètres et 700 nanomètres, de préférence compris entre 380 nanomètres et 450 nanomètres.

13. Utilisation du procédé de détermination selon l'une quelconque des revendications **1** à **12,** dans un procédé de suivi d'un traitement contre une maladie liée à l'hème ou à une hémoprotéine chez un sujet souffrant de la maladie liée à l'hème ou à une hémoprotéine et ayant reçu le traitement, le procédé comprenant au moins les étapes consistant à :

- effectuer les étapes d'un procédé de détermination au moins une teneur en protéine dans un échantillon biologique du sujet, pour obtenir des teneurs déterminées en protéine, le procédé de détermination étant selon l'une quelconque des revendications **1** à **12,** et
- suivre la teneur déterminée en protéines pour suivre un traitement contre une maladie liée à l'hème ou à une hémoprotéine chez un sujet souffrant de la maladie liée à l'hème ou à une hémoprotéine et ayant reçu le traitement.

14. Programme d'ordinateur comportant des instructions pour l'exécution des étapes d'un procédé ou une utilisation selon l'une quelconques des revendications **1** à **13** lorsque ledit programme est exécuté par un ordinateur.


**Patentansprüche**

1. Verfahren zur Bestimmung eines Gehalts mindestens eines Proteins, das in einer biologischen Flüssigkeit vorliegt, umfassend die Schritte:

a) Erhalten eines Absorptionsspektrums SA einer Probe der biologischen Flüssigkeit;
b) Subtrahieren einer Spektrumkomponente, die mit Oxyhämoglobin assoziiert ist, von dem Absorptionsspektrum SA, um ein berechnetes Zwischenspektrum SC1 zu erhalten; wobei die Spektrumkomponente, die mit Oxyhämoglobin assoziiert ist, durch Normalisierung eines Referenzspektrums SR1 von Oxyhämoglobin erhalten wird, wobei die Normalisierung in Bezug auf das Absorptionsspektrum SA durchgeführt wird, indem ein Normalisierungskoeffizient K1 bei einer Wellenlänge berechnet wird, die aus der Gruppe bestehend aus 416 Nanometer, 543 Nanometer und 577 Nanometer ausgewählt ist;
c) Subtrahieren einer Spektrumkomponente, die mit Methämoglobin assoziiert ist, von dem berechneten Zwischenspektrum SC1, um ein berechnetes Zwischenspektrum SC2 zu erhalten; wobei die Spektrumkomponente, die mit Methämoglobin assoziiert ist, aus mindestens einem Spektrum erhalten wird, das aus dem Absorptionsspektrum SA, dem Absorptionsspektrum der Probe in Gegenwart einer Zugabe von KCN, dem Referenzspektrum des Methämoglobins (SR2) und dem Referenzspektrum des Methämoglobins (SR2) in Gegenwart von KCN ausgewählt ist;
d) Subtrahieren einer Spektrumkomponente, die mit Bilirubin assoziiert ist, von dem berechneten Zwischenspektrum SC2, um ein berechnetes Zwischenspektrum SC3 zu erhalten; wobei die Spektrumkomponente, die mit Bilirubin assoziiert ist, durch Normalisierung eines Referenzspektrums SR3 von Bilirubin erhalten wird, wobei die Normalisierung in Bezug auf das berechnete Zwischenspektrum SC2 durchgeführt wird, indem ein Normalisierungskoeffizient K3 in einem Bereich von Wellenlängen berechnet wird, der zwischen 200 Nanometer und 800 Nanometer liegt, vorzugsweise zwischen 350 Nanometer und 700 Nanometer liegt;
e) Subtrahieren einer Spektrumkomponente, die mit Häm, das mit Serumalbumin verbunden ist (Häm-SA), assoziiert ist, von dem berechneten Zwischenspektrum SC3, um ein berechnetes Zwischenspektrum SC4 zu erhalten; wobei die Spektrumkomponente, die mit Häm-SA assoziiert ist, durch Normalisierung eines Referenzspektrums SR4 von Häm-AS erhalten wird, wobei die Normalisierung in Bezug auf das berechnete Zwischenspektrum SC3 durchgeführt wird, indem ein Normalisierungskoeffizient K4 bei einer Länge in einem Bereich von Wellenlängen berechnet wird, der zwischen 200 Nanometer und 800 Nanometer liegt, vorzugsweise zwischen 300 Nanometer und 700 Nanometer liegt, vorzugsweise zwischen 380 Nanometer und 450 Nanometer liegt; und gegebenenfalls

f) Subtrahieren einer Spektrumkomponente, die mit mindestens einem anderen Hämprotein assoziiert ist, das in der biologischen Flüssigkeit vorliegt, von dem berechneten Zwischenspektrum SC4, um ein berechnetes Zwischenspektrum SC5 zu erhalten; wobei die Spektrumkomponente, die mit mindestens einem anderen Hämprotein assoziiert ist, durch Normalisierung eines Referenzspektrums SR5 des mindestens einen anderen Hämproteins erhalten wird; wobei die Normalisierung in Bezug auf das berechnete Zwischenspektrum SC5 durchgeführt wird, indem ein Normalisierungskoeffizient K5 in einem Bereich von Wellenlängen berechnet wird, der zwischen 200 Nanometer und 700 Nanometer liegt.

2. Verfahren nach Anspruch **1,** wobei das Spektrum vom Schritt a) in einem Bereich von Wellenlängen, der das gesamte oder einen Teil des Ultraviolettlichts und das gesamte oder einen Teil des sichtbaren Lichts umfasst, vorzugsweise in einem Bereich von Wellenlängen, die von 200 Nanometer bis 800 Nanometer reichen, vorzugsweise von 300 Nanometer bis 700 Nanometer reichen, erhalten wird.

3. Verfahren nach Anspruch **1** oder **2,** wobei das Spektrum vom Schritt a) von einer Probe der verdünnten biologischen Flüssigkeit erhalten wird, um eine maximale optische Dichte des Absorptionsspektrums SA zu erhalten, die kleiner als oder gleich 2, vorzugsweise kleiner als oder gleich 1 ist.

4. Verfahren nach einem der Ansprüche **1** bis **3,** wobei die Probe der biologischen Flüssigkeit auf einen pH-Wert, der kleiner als 8 ist, vorzugsweise auf einen pH-Wert, der zwischen 7,35 und 7,45 liegt, vorzugsweise gleich 7,4 ist, gepuffert wird.

5. Verfahren nach einem der Ansprüche **1** bis **4,** wobei der Normalisierungskoeffizient K1 durch Bestimmung des Verhältnisses zwischen den Werten von zweiten Ableitungen des Absorptionsspektrums und des Referenzspektrums SR1 bei einer der Wellenlängen der Gruppe berechnet wird.

6. Verfahren nach einem der Ansprüche **1** bis **5,** wobei der Normalisierungskoeffizient K1 bei einer Wellenlänge von 577 Nanometer berechnet wird.

7. Verfahren nach einem der Ansprüche **1** bis **6,** wobei das Oxyhämoglobin mindestens eines von Oxyhämoglobin A und Oxyhämoglobin F ist.

8. Verfahren nach einem der Ansprüche **1** bis **7,** wobei der Schritt c) durch Subtrahieren der Spektrumkomponente, die mit Methämoglobin assoziiert ist und die durch Normalisierung eines Referenzspektrums SR2 des Methämoglobins erhalten wird, umgesetzt wird, wobei die Normalisierung in Bezug auf das berechnete Zwischenspektrum SC1 durchgeführt wird, indem ein Normalisierungskoeffizient K2 bei einer Wellenlänge berechnet wird, die zwischen 350 Nanometer und 700 Nanometer liegt, vorzugsweise zwischen 380 Nanometer und 440 Nanometer liegt.

9. Verfahren nach einem der Ansprüche **1** bis **8,** wobei der Normalisierungskoeffizient K3 durch Bestimmung des Verhältnisses zwischen den Werten von zweiten Ableitungen des Absorptionsspektrums SC2 und des Referenzspektrums SR3 bei einer Wellenlänge berechnet wird, die zwischen 350 Nanometer und 700 Nanometer liegt.

10. Verfahren nach einem der Ansprüche **1** bis **9,** wobei der Normalisierungskoeffizient K4 durch Bestimmung des Verhältnisses zwischen den Werten von zweiten Ableitungen des Absorptionsspektrums und des Referenzspektrums SR4 bei einer Wellenlänge berechnet wird, die von ungefähr 350 nm bis ungefähr 460 nm reicht.

11. Verfahren nach einem der Ansprüche **1** bis **10,** wobei das mindestens eine andere Hämprotein, das in der biologischen Flüssigkeit vorliegt, aus Häm, das mit Hämopexin verbunden ist, carboxyliertem Hämoglobin, einem Biomarker des Katabolismus von Häm durch Hämoxygenase, Myoglobin, Porphyrinen, Abbauprodukten von Bilirubin, Porphobilin von Urinen, Sterkobilin und Urobilin ausgewählt ist.

12. Verfahren nach einem der Ansprüche **1** bis **11,** wobei das mindestens eine andere Hämprotein, das in der biologischen Flüssigkeit vorliegt, Häm, das mit Hämopexin verbunden ist (Häm-Hx), ist und wobei die Schritte e) und f) kombiniert werden, indem der Schritt e') durch Subtrahieren der Spektrumkomponente, die mit der linearen Kombination von Häm-SA und Häm-Hx assoziiert ist, umgesetzt wird, wobei die Spektrumkomponente durch Normalisierung eines Referenzspektrums SR4' des Beitrags von zwei Referenzspektren SR4 von Häm-AS und SR5 von Häm-Hx erhalten wird, wobei die Normalisierung in Bezug auf das berechnete Zwischenspektrum SC3 durchgeführt wird, indem zwei Normalisierungskoeffizienten K4 und K5 bei einer Wellenlänge berechnet werden, die einen Bereich von Wellenlängen umfasst, der zwischen 200 Nanometer und 800 Nanometer liegt, vorzugsweise zwischen 300 Nanometer und 700 Nanometer liegt, vorzugsweise zwischen 380 Nanometer und 450 Nanometer liegt.

13. Verwendung des Bestimmungsverfahrens nach einem der Ansprüche **1** bis **12** in einem Verfahren zur Überwachung einer Behandlung einer Krankheit, die mit Häm oder mit einem Hämoprotein verbunden ist, bei einem Probanden, der an der Krankheit, die mit Häm oder mit einem Hämoprotein verbunden ist, leidet und die Behandlung erhalten hat, wobei das Verfahren mindestens die Schritte umfasst, bestehend aus:

    - Durchführen der Schritte eines Verfahrens zur Bestimmung mindestens eines Proteingehalts in einer biologischen Probe des Probanden, um bestimmte Proteingehalte zu erhalten, wobei das Bestimmungsverfahren nach einem der Ansprüche **1** bis **12** ist, und
    - Überwachen des bestimmten Gehalts von Proteinen durch Überwachen einer Behandlung einer Krankheit, die mit Häm oder mit einem Hämoprotein verbunden ist, bei einem Probanden, der an der Krankheit, die mit Häm oder mit einem Hämoprotein verbunden ist, leidet und die Behandlung erhalten hat.

14. Computerprogramm, umfassend Anweisungen zur Ausführung von Schritten eines Verfahrens oder einer Verwendung nach einem der Ansprüche **1** bis **13,** wenn das Programm von einem Computer ausgeführt wird.

**Claims**

1. A process for determining the content of at least one protein present in a biological fluid comprising the steps of:

    a) obtaining an SA absorption spectrum from a sample of the biological fluid;
    (b) subtracting from the absorption spectrum SA a spectral component associated with oxyhaemoglobin in order to obtain a calculated intermediate spectrum SC1; said spectral component associated with oxyhaemoglobin being obtained by normalizing an SR1 reference spectrum of oxyhaemoglobin, said normalisation being carried out with respect to said absorption spectrum SA by calculating a normalization coefficient K1 at a wavelength chosen from the group consisting of 416 nanometers, 543 nanometers and 577 nanometers;
    (c) subtracting from the intermediate calculated spectrum SC1 a spectral component associated with methemoglobin in order to obtain a calculated intermediate spectrum SC2; said spectral component associated with methemoglobin being obtained from at least one spectrum chosen from the SA absorption spectrum, the absorption spectrum of the sample in the presence of an addition of KCN, the methemoglobin reference spectrum (SR2) and the methemoglobin reference spectrum (SR2) in the presence of KCN;
    (d) subtracting from said calculated intermediate SC2 spectrum a bilirubinassociated spectral component in order to obtain a calculated intermediate SC3 spectrum; said spectral component associated with bilirubin being obtained by normalization of a reference spectrum SR3 of bilirubin, said normalization being carried out with respect to said calculated intermediate spectrum SC2 by calculating a normalization coefficient K3 in a wavelength range between 200 nanometers and 800 nanometers, preferably between 350 nanometers and 700 nanometers;
    (e) subtracting from said calculated intermediate SC3 spectrum a spectral component associated with serum albumin-bound heme (heme-AS) in order to obtain a calculated intermediate SC4 spectrum; said spectral component associated with heme -AS being obtained by normalization of a reference spectrum SR4 of heme-AS, said normalization being performed with respect to said calculated intermediate spectrum SC3 by calculating a normalization coefficient K4 at a wavelength in a wavelength range between 200 nanometers and 800 nanometers, preferably between 300 nanometers and 700 nanometers, preferably between 380 nanometers and 450 nanometers; and possibly
    (f) subtracting from the calculated intermediate SC4 spectrum a spectral component associated with at least one other heme protein present in the biological fluid in order to obtain a calculated intermediate SC5 spectrum; said spectral component associated with said at least one other heme protein being obtained by normalizing an SR5 reference spectrum of said at least one other heme protein; said normalization being carried out with respect to said calculated intermediate spectrum SC5 by calculating a normalization coefficient K5 in a wavelength range between 200 nanometers and 700 nanometers.

2. The process of claim **1,** wherein the spectrum of step (a) is obtained in a wavelength range comprising all or part of the ultraviolet and all or part of the visible, preferably in a wavelength range from 200 nanometers to 800 nanometers, preferably from 300 nanometers to 700 nanometers.

3. The process of claim **1** or **2,** wherein the spectrum of step (a) is obtained from a diluted sample of the biological fluid, in order to obtain a maximum optical density of the absorption spectrum SA less than or equal to 2, preferably less than or equal to 1.

4. The process according to any one of claims **1** to **3,** wherein the sample of the biological fluid is buffered to a pH of less than 8, preferably to a pH of 7.35 to 7.45, preferably of 7.4.

5. The process according to any one of claims **1** to **4,** wherein the normalization coefficient K1 is calculated by determining the ratio between the values of the second derivatives of the absorption spectrum and the reference spectrum SR1 at one of the wavelengths of the group.

6. The process according to any one of claims **1** to **5,** wherein the normalization coefficient K1 is calculated at a wavelength of 577 nanometers.

7. The process according to any one of claims **1** to **6,** wherein the oxyhaemoglobin is at least one of oxyhaemoglobin A and oxyhaemoglobin F.

8. The process according to any one of claims **1** to **7,** wherein step (c) is performed by subtracting the spectral component associated with the methemoglobin, obtained by normalizing a reference spectrum SR2 of methemoglobin, said normalization being performed with respect to said calculated intermediate spectrum SC1 by calculating a normalization coefficient K2 at a wavelength between 350 nanometers and 700 nanometers, preferably between 380 nanometers and 440 nanometers.

9. A process according to any one of claims **1** to **8,** wherein the normalization coefficient K3 is calculated by determining the ratio between the values of the second derivatives of the absorption spectrum SC2 and the reference spectrum SR3 at a wavelength between 350 nanometers and 700 nanometers.

10. The process according to any one of claims **1** to **9,** wherein the normalization coefficient K4 is calculated by determining the ratio between the values of the second derivatives of the absorption spectrum and the reference spectrum SR4 at a wavelength ranging from about 350 nm to about 460 nm.

11. A process according to any one of claims **1** to **10,** wherein said at least one other heme protein present in the biological fluid is selected from hemepexin-bound hememe, carboxylated haemoglobin, a biomarker of heme catabolism by heme oxygenase, myoglobin, porphyrins, bilirubin breakdown products, urine porphobilin, stercobilin and urobilin.

12. A process according to any one of claims **1** to **11,** wherein said at least one other protein present in the biological fluid is hemopexin-bound heme (heme-Hx), wherein steps (e) and (f) are combined in step (e') being performed by subtracting the spectral component associated with the linear combination of heme-AS and heme-Hx, said spectral component being obtained by normalizing a reference spectrum SR4' of the contribution of two reference spectra SR4 of heme-AS and SR5 of heme-Hx, said normalization being carried out with respect to said calculated intermediate spectrum SC3 by calculating two normalization coefficients K4 and K5 at a wavelength range between 200 nanometres and 800 nanometres, preferably between 300 nanometers and 700 nanometers, preferably between 380 nanometers and 450 nanometers.

13. Use of the process for determining according to any one of claims **1** to **12** in a process of monitoring treatment of a heme- or haemoprotein-related disease in a subject suffering from the heme- or haemoprotein-related disease and who has received the treatment, the process comprising at least the steps of:

   - performing the steps of a process for determining at least a protein content in a biological sample of the subject, to obtain specified protein contents, the process of determination being according to any one of claims **1** to **12,** and
   - monitoring the determined protein content in order to monitor the treatment of a heme- or haemoprotein-related disease in a subject suffering from the heme- or haemoprotein-related disease that has received the treatment.

14. A computer program with instructions for performing the steps of a process or the use according to any one of claims **1** to **13** when said program is executed by a computer.

# FIGURE 1

## Plasma DHTR

1ère étape : 1er spectre du plasma
2ème étape: plasma - HbO2
3ème étape: plasma - HbO2 - Bilirubine
4ème étape: plasma - HbO2 - Bilirubine - MetHb (2nd spectre + KCN)
5ème étape: plasma - HbO2 - Bilirubine - MetHb - HSA-heme

dosage de l'hème avec ApoHb

. Hb = 11 mg/dL avec 18 % Fe3+
(normale < 4 mg/dL)

. bilirubine totale 75 µM (4.4 mg/dL)
(normale < 1.2 mg/dL)

. heme = 11 µM
(normale < 0.2 µM)

HSA-heme

4th

produits d'oxydation (bilirubine/heme)

**FIGURE 2a**

FIGURE 2b

**FIGURE 2c**

**FIGURE 2d**

**FIGURE 3a**

**FIGURE 3b**

FIGURE 4

EP 3 717 903 B1

EP 3 717 903 B1

**FIGURE 5a**

FIGURE 5b

EP 3 717 903 B1

FIGURE 5c

EP 3 717 903 B1

**FIGURE 5d**

## FIGURE 6

| | Nomenclature | Signification |
|---|---|---|
| 1 | SA | Spectre d'absorption |
| 2 | $SA^{KCN}$ | Spectre d'absorption avec KCN |
| 3 | SR | Spectre de référence |
| 4 | SC | Spectre calculé |
| 5 | $SDR^{KCN}$ | Spectre différentiel de référence avec KCN |
| 6 | K | Coefficient de normalisation |
| 7 | $d^2$ | Dérivée seconde |
| 8 | $\lambda$ | Longueur d'onde |
| 9 | AS | Albumine sérique |
| 10 | HAS | Albumine sérique humaine |

EP 3 717 903 B1

**EP 3 717 903 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **H J DUISER et al.** itérative model for the calculation of oxyhemoglobin, methemoglobin, and bilirubin in absorbance spectra of cerebrospinal fluid. *Clinical Chemistry,* 01 Février 2001, 338-341